# EUROPÄISCHE PATENTSCHRIFT

(11) **EP 1 834 613 B1**
(45) Veröffentlichungstag und Bekanntmachung des Hinweises auf die Patenterteilung: **05.12.2012**
(21) Anmeldenummer: 07104043.0
(22) Anmeldetag: 13.03.2007
(51) Int. Cl.: A61F 5/02

(54) **Baukasten für Wirbelsäulenorthesen**
Kit for spinal ortheses
Jeu de construction pour orthèses de la colonne vertébrale

(30) Priorität: 14.03.2006 DE 202006004190 U; 15.09.2006 DE 102006043846
(43) Veröffentlichungstag der Anmeldung: 19.09.2007
(73) Patentinhaber: Zours, Claudia, 44797 Bochum (DE)
(72) Erfinder: Zours, Claudia, 44797 Bochum (DE)
(74) Vertreter: Bals, Rüdiger

(56) Entgegenhaltungen:
- WO-A-00/15164
- WO-A-03/075807
- DE-A1- 10 329 454
- GB-A- 740 507

## Beschreibung

Die vorliegende Erfindung liegt auf dem Gebiet der Wirbelsäulenorthesen, die aus Elementen eines Baukastensystems konfiguriert sind.

Beispielsweise sind derartige Wirbelsäulenorthesen unter anderem aus der deutschen Offenlegungsschrift DE 103 29 454 A1 sowie der Druckschrift WO 00/15164 bekannt.

Unterschiedliche Beschwerden im Wirbelsäulenbereich werden mit unterschiedlichen Orthesen behandelt, welche die Wirbelsäule in den betroffenen Abschnitten stützt, aufrichtet und/oder ihre Beweglichkeit in den unterschiedlichen Ebenen einschränkt. Das Stützverhalten und die Steifigkeit der Orthese wird vorgegeben von dem Genesungsfortschritt des Patienten. In der Vergangenheit wurden im Verlauf der Genesung immer wieder neue Orthesen verordnet, deren Stützverhalten oder Steifigkeit der Indikation ebenso wie dem gegenwärtigen Genesungsfortschritt des Patienten Rechnung trägt.

Um die Kosten für die Krankenversicherer zu senken, wurden Baukastensysteme für Orthesen entwickelt, die in gewissem Maße grob auf den zu stützenden Wirbelsäulenbereich anpassbar sind. Ein Beispiel für ein derartiges Baukastensystem ist aus der DE 103 29 454 A1 bekannt.

Die vorbekannten Baukastensysteme aus DE 103 29 454 A1 und WO 00 / 15164 genügen jedoch nicht allen Anforderungen an Flexibilität und Anpassungsfähigkeit. So erlauben die bekannte Baukastensysteme nicht die wahlweise Abstützung in Sagittalebene oder die gleichzeitige Abstützung in Sagittal- und Frontalebene. Weiter ist nicht vorgesehen, die Abstützungssteifigkeit an den jeweiligen Genesungsforschritt anzupassen. Schließlich erlaubt die genannten Baukastensystem keine feinschrittige Anpassung der daraus zu konfigurierenden Orthese an die jeweilige Körpergröße des Trägers. Aus der WO-A-03/075807 ist ein Stützrahmen Zu Entlastung der Wirbelsäule bekannt.

Der vorliegenden Erfindung liegt daher die Aufgabe zugrunde, ein Baukastensystem für Wirbelsäulenorthesen anzugeben, dessen Wirbelsäulenorthesen in einem hohen Maße flexibel an die jeweiligen Beschwerden und den Genesungsfortschritt des Patienten anpassbar sind. Mit einer möglichst geringen Anzahl von Elementen soll eine möglichst große Vielzahl von Orthesen konfigurierbar sein, die bei unterschiedlichen Leiden einsetzbar sind und deren Abstützungseigenschaften sukzessive auf- bzw. abgebaut werden kann.

Gelöst wird diese Aufgabe durch ein Baukastensystem, welches die technischen Merkmale aus dem Anspruch 1 aufweist.

Die in Anspruch 1 beschriebenen sieben Elemente (siehe auch a1) bis c1),e1), g1), h1) und i1)) - das untere Bauchmieder (auch Lendenwirbelsäulen-Mieder genannt), das obere Bauchmieder (auch Trageriemensystem genannt), das Miederstützelement (auch Basispelotte genannt), das Brustwirbelsäulenmieder (auch BWS-Orthese oder Thorax-Orthese genannt), die bogenförmige Stützspange an einem BWS-Orthese oder Thorax-Orthese genannt), die bogenförmige Stützspangen an einem Miederstützelement, das Stützelement sowie die Bauchpelotte - stellen das Grundbaukastensystems dar, aus dem die erfindungsgemäßen Orthesen konfigurierbar sind, Einige Grundelemente des Bausatzes werden für unterschiedliche Orthesen benötigt und sind somit weiterverwertbar, wenn der Patient eine andere Orthese benötigt. Auch erlaubt das Baukastensystem ein therapeutisch korrektes Abschulen, in dem aus einer komplexeren Orthese sukzessiv Elemente entnommen werden und diese dadurch schrittweise abgebaut wird. Es ist jederzeit möglich, bei Genesungsverzögerungen die verfrüht abgebaute Orthese zu einer höheren Ausbaustufe zurückzubauen. Schutzgegenstand ist jede Wirbelsäulenorthese nach Anspruch 1, die aus dem erweiterten Baukastensystem nach Anspruch 2 konfigurierbar ist, auch wenn die Wirbelsäulenorthese Prinzip bedingt nicht von allen Elementen des erweiterten Baukastensystems Gebrauch machen kann.

Der Vorteil bei diesem grundlegenden Baukastensystem ist darin zu sehen, dass mit einer minimalen Anzahl von Elementen, wie sie Im Anspruch 1 und 2 aufgezählt sind, die unterschiedlichsten Orthesen für den jeweiligen Anwendungsfall zusammenstellbar sind. Dabei lassen sich die jeweiligen Orthesen passgenau und flexibel an den jeweiligen medizinischen und körperlichen Bedarf des Patienten anpassen.

Optional kann ein Schultergurtsystem unelastische Gurte enthalten, die insbesondere ein Kunststofftextil aufweisen. Ferner ist es denkbar, dass das Schultergurtaystem über Gurthalter am Rückenteil angeordnet ist, wobei die Gurthalter, insbesondere über elastische Bänder (16), unlösbar an dem Rückenteil (11) befestigt sind.

Zweckmäßig Ausgestaltungen des ersten grundlegenden Baukastensystems einer Wirbelsäulenorthese sind in den Unteransprüchen 2 bis 9 aufgeführt.

Der wesentliche Unterschied zum zweiten grundlegenden Ausführungebeispiel aus den weiteren Ansprüchen 10 bis 15 ist darin zu sehen, dass zusätzliche Elemente hinzugenommen werden müssen, um die jeweiligen Orthesen untereinander erweiterbar auszugestalten. Beispielsweise kommt im zweiten grundlegenden Ausführungsbeispiel ein zusätzliches Verbindungselement zum Einsatz. Außerdem sind die Miederstützelemente aufwendig als Pelotten ausgestaltet. In dem zweiten grundiegenden Ausführungsbeispiel kommt als Miederstützelemente eine Basispelotte zum Einsatz.

Die in Anspruch 10 beschriebenen neun Elemente (siehe hierzu a2 bis i2)) - das untere Bauchmleder, das obere Bauchmieder, die Basispelotte, die Lumbalerweiterung für die Basispelotte, das Brustwirbelsäulenmieder, das Verbindungselement, der Überbrückungsrahmen, das Stützelement und die Bauchpelotte - stellen die Basiselemente des weiteren Baukastensystems dar, aus dem erfindungsgemäß mindestens sieben unterschiedliche Orthesen konfigurierbar sind. Einige Grundelemente des Bausatzes werden für unterschiedliche Orthesen benötigt und sind somit weiterverwertbar, wenn der Patient eine andere Orthese benötigt. Auch erlaubt das Baukastensystem ein therapeutisch korrektes Abschulen, in dem aus einer komplexeren Orthese sukzessiv Elemente entnommen werden und diese dadurch schrittweise abgebaut wird, Es ist jederzeit möglich, bei Genesungsverzögerungen die verfrüht abgebaute Orthese zu einer höheren Ausbaustufe zurückzubauen.

Zweckmäßigerweise wird als Basispelotte eine für sich bekannte Gliederpelotte gewählt, umfassend vier gelenkig miteinander verbindbare Glieder, welche entlang der Lendenwirbelsäule angeordnet sind und den dritten bis zum fünften Lendenwirbel, sowie den ersten Kreuzwirbel transversal überbrücken, Eine derartige Gliederpelotte hat sich in der Theraplepraxis gegenüber anderen Pelotton hervorragend bewährt.

Da die Gelenke der Gliederpelotten in der Regel auch eine Beweglichkeit der einzelnen Glieder untereinander In Sagittalebene erlauben, empfiehlt es sich, innerhalb des Baukastensystems ein Fixierelement vorzusehen, das posterior auf die Gliederpelotte aufgesetzt werden, so dass die Glieder der Gliederpelotte in Sagittalebene unbeweglich untereinander fixiert werden.

Eine geeignete Lumbalerweiterung für eine Gliederpelotte umfasst zwei gelenkig miteinander verbindbare Glieder sowie zwei Stützschienen, wobei die Glieder der Lumbalerweiterung in die Gliederpelotte dergestalt einsetzbar sind, dass die Glieder der um die Lumbalerweiterung erweiterte Gliederpelotte den ersten bis zum fünften Lendenwirbel, sowie den ersten Kreuzwirbel transversal überbrücken, und wobei die Stützschienen lateral auf die Gliederpelotte dergestalt aufsetzbar sind, dass zumindest die Glieder, welche die ersten vier Lendenwirbel überbrücken, unbeweglich untereinander fixiert sind. Die Lumbalerweiterung für eine Gliederpelotte wird also nicht an die bestehende Gliederpelotte angehängt, um die Wirbel L1 und L2 mit zu erfassen, sondern in die Gliederpelotte eingesetzt, sodass das Glied, das in der Basiskonfiguration dem L3 zugeordnet war, nun den L1 überdrückt. Der L2 erhält das eine Glied der Lumbalerweiterung, das andere Glied der Lumbalerweiterung stützt fortan den L3.

Um die Abstützungssteifigkeit des Überbrückungsrahmens besser einstellbar zu gestalten, empfiehlt es sich, ein zehntes Element In Gestalt von Versteifungsstäben vorzusehen, die zur Einstellung der Steifigkeit an dem Überbrückungsrahrmen festlegbar sind.

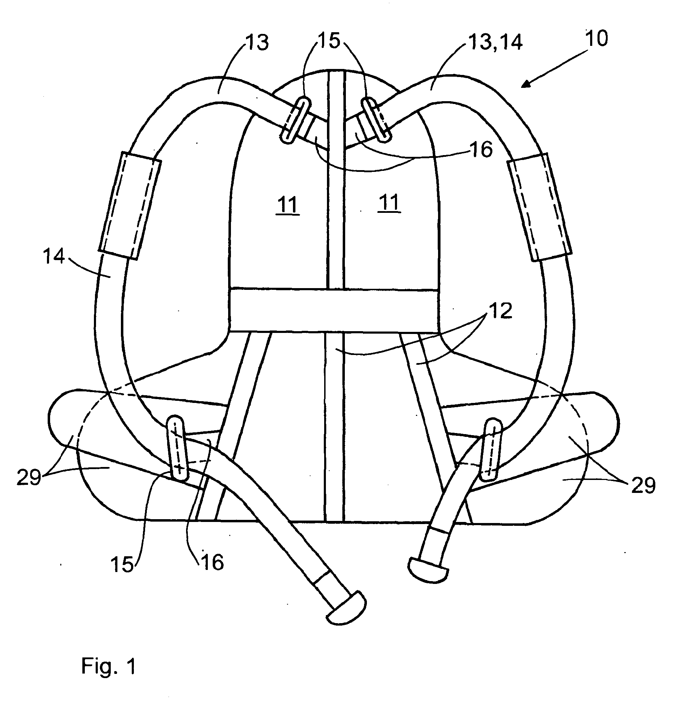

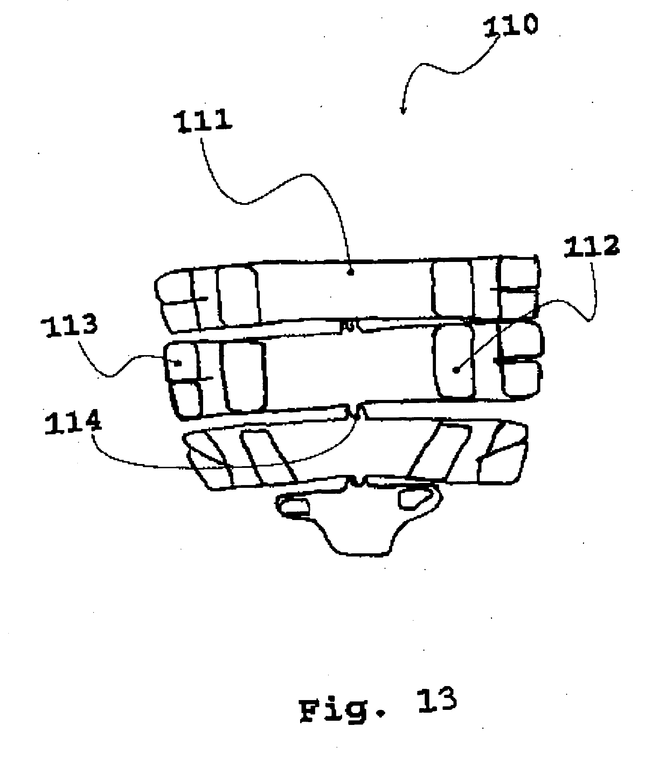

Weltere, die Erfindung verbessernde Maßnahmen sind in den Unteransprüchen angegeben oder werden nachfolgend gemeinsam mit der Beschreibung und den bevorzugten Ausführungebeispielen der Erfindung anhand der Figuren näher dargestellt, In den Figuren 1 bis 12 ist das erste grundlegende Baukastensystem für. Wirbelsäulenorthesen gezeigt. Die weiteren Figuren 13 bis 28 offenbaren dagegen das weitere grundlegende Baukastensystem für Wirbelsäulenorthesen. Die erfindungsgemäße Wirbelsäulenorthese in beiden Baukastensystemen ist hierbei mindestens aus unteren Bauchmieder, einem oberen Bauchmieder und einem Brustwirbelsäulenmieder aufgebaut. Es zeigen:
- Fig. 1: eine Rückansicht einer Brustwirbelsäulen-Orthese ohne Tragriemensystem,
- Fig. 2: Vorderansicht auf eine Brustwirbalsäulen-Orthese mit einem Tragrlemensystem und einem bügelförmigen Stützelement,
- Fig. 3a: Vorderansicht auf ein bügelförmiges Stützelement,
- Fig. 3b: Seitenansicht des Stützelementes aus Fig. 3a,
- Fig. 3c: Querschnitt I-I durch das Stützelement aus Fig. 3a,
- Fig. 7: dreidimensionale Ansicht eines zusammengefügten Stützelementes mit einem Miederstützelement, wobei zwei bogenförmige Stützspangen angeordnet sind,
- Fig. 8: dreidimensionale Ansicht eines Adapterteils,
- Fig. 9: dreidimensionale Ansicht einer erfindungsgemäßen Wirbelsäulen-Orthese ohne bogenförmige Stützspange,
- Fig. 10: dreidimensionale Ansicht einer Thorako-Lumbal-Orthese,
- Fig. 11: dreidimensionale Ansicht eines Lendenwirbelsäulen-Mieders und
- Fig. 12a: Vorderansicht auf einen Gurthalter und
- Fig. 12b: Schnitt II-II durch den Gurthalter aus Fig. 12a.
- Fig. 13: Gliederpelotte;
- Fig. 14: Stabilisierungsorthese;
- Fig. 15: Gliederpelotte mit Lumbalerweiterung;
- Fig. 16: Lumbalorthese;
- Fig. 17: Fixierelement;
- Fig.18: Stützelement;
- Fig.19: Verbindungselement;
- Fig. 20: Gliederpelotte mit Lumbalerweiterung, Verbindungselement und Stützelement;
- Fig. 21: Thoraxorthese;
- Fig. 22: Wirbelsäulenorthese sagittal;
- Fig. 23: Stützrahmen mit Versteifungsstäben;
- Fig. 24: Bauchpelotte;
- Fig. 25: Überbrückungsorthese:
- Fig. 26: Flexionsorthese;
- Fig. 27: Überbrückungsrahmen mit Stützelementen;
- Fig. 28: Osteoporoseorthese

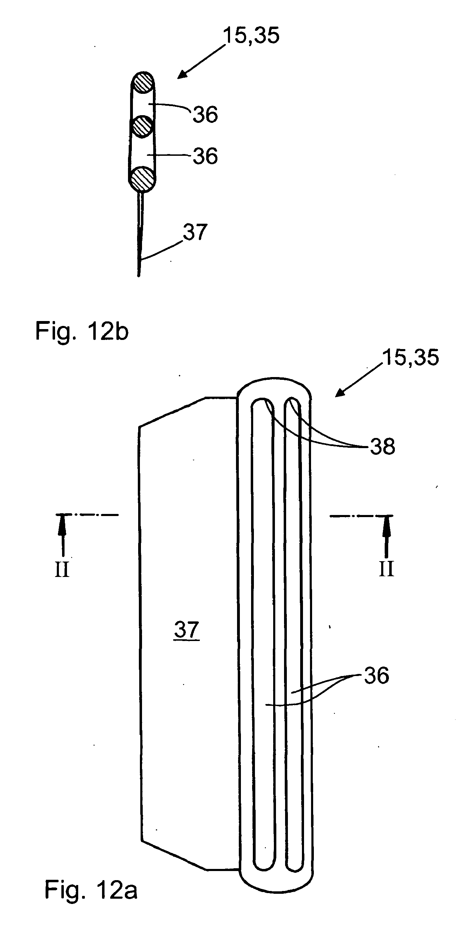

In der Fig. 1 ist eine erfindungsgemäße BWS-Orthese 10 in Rückansicht dargestellt. Dabei ist das dreiecksförmige Rückenteil 11 mit den stark abgerundeten Ecken und zusätzlichen streifenförmigen Bahnen 12 in Form von Stoffbahnen oder Klettverschllusstellen erkennbar. Dieses Rückenteil 11 ist spiegelsymmetrisch zur Klettverschlusstellen erkennbar. Dieses Rückenteil 11 ist spiegelsymmetrisch zur Wirbelsäule bzw. zu einer vertikalen Linie aufgebaut. Am kranialen Ende des Rückenteils 11 sind zwei unelastische Gurte 14 des Schultergurtsystems 13 im Bereich der Wirbelsäule angeordnet. Dabei werden Gurthalter 15 verwendet, die im Wesentlichen aus einem geschlossenen bogenförmigen Kunststoffteil bestehen. Diese Gurthalter 16 sind über elastische Bänder 16 an dem Rückenteil 11 befestigt. Dabei verlaufen die elastischen Bänder 16 diagonal von der Wirbelsäule weg, wobei die Bänder zur Schulter hin aufsteigend angeordnet sind. An den Gurthaltern 15 sind die unalastischen Gurte 14 des Schultergurtsystems 13 angeordnet. Diese verlaufen zu den unteren Gurthaltern 15, die ebenfalls über elastische Bänder 16 an dem Rückenteil 11 angeordnet sind. Um den Schultergurtsystem 13 nicht zu viel Spielraum durch die elastischen Bänder 16 zu geben, sind die unteren Gurthalter 15 weiter weg von der Wirbelsäule am Rückenteil 11 angeordnet. Somit werden nur kurze elastische Bänder 16 verwendet, die beispielsweise mit dem Rückenteil 11 vernäht oder verklebt sein können. Die kaudalen Gurthalter 15 weisen Umlenkschnallen 35 auf, die in der Fig. 12a und 12b separat dargestellt sind.

Zur ventralen Befestigung der BWS-Orthese 10 ist ein Tragriemensystem 17 vorgesehen, welches Riemen 18 enthält, die in der Fig. 1 nicht eingezeichnet sind. Dieses Tragrlemensystem 17 kann zum Beispiel durch die - in der Fig. 1 dargestellten - doppelwandigen Befestigungslappen 29 an dem Rückenteil 11 befestigt werden. Hierzu sind die Befestigungslappen 29 auf ihrer Innenseite mit entsprechenden Klettverschlussteilen versehen, die mit Gegenklettverschlyssteilen an den Riemen 18 des Tragrlemensystems 17 zusammenwirken können. Dabei werden die Riemen 18 über die stationären Riemenenden 19 an dem Befestigungslappen 29 angeordnet. Um die Riemen 18 auf die notwendige Länge zu kürzen, ist es möglich, einfach die stationären Riemenenden 19 entsprechend abzuschneiden. Das Tragriemensystem 17 wird Ober die offenen Riemenenden 20 zum Beispiel mit einem Klettverschlussteil ventral verschlossen. Ein vorgesehenes Stützelement 23 ist vorzugsweise auf der zum Rücken zugewandten Seite des Rückenteils 11 angeordnet und damit In der Fig. 1 nicht sichtbar.

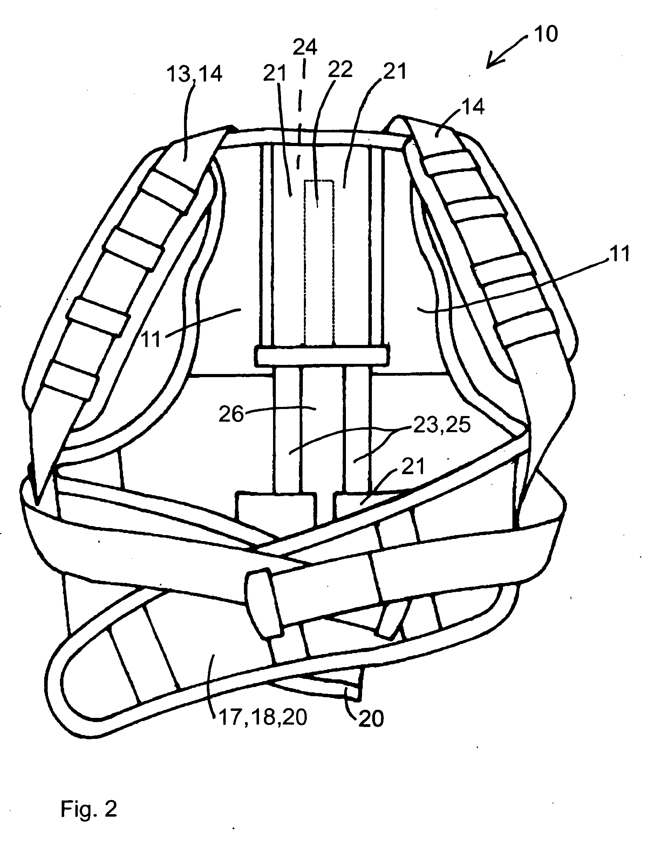

In der Fig. 2 ist eine weitere nicht selbständig beanspruchte BWS-Orthese 10 gezeigt. Dabei ist deutlich, dass innerhalb der Taschen 21 angeordnete Stützelement 23 zu erkennen. Dieses Stützelement 23 ist bügelförmig, insbesondere "U"-förmig, wobei das "U" auf dem Kopf steht, ausgestaltet. Sowohl das kraniale Ende 24 des Stützelementes 23 als auch die kaudalen Enden 25 des Stützelementes 23 sind in verschließbaren Taschen 21 angeordnet. Diese Taschen 21 lassen sich im vorliegenden Fall durch Klettverschlüsse 22 schließen. Bei der dargestellten BWS-Orthese 10 aus Fig. 2 ist das Tragriemensystem 17 unlösbar mit dem Rückenteil 11 verbunden. Folglich kommen die Befestigungslappen 29 zur Aufnahme des Tragrlemensystems 17, insbesondere der Riemen 18, nicht zum Einsatz. Wie weiter deutlich zu erkennen ist, weist das Stützelement 23 eine Ausnehmung 26 zwischen den beiden stabähnlichen Enden 25 auf, um die Wirbelsäule in dieser Ausnehmung aufnehmen zu können. Diese nicht selbstständig beanspruchte BWS-Orthese 10 ist in dem dargestellten Zustand aus Fig. 2 voll einsatzfähig. Selbstverständlich können auch weitere Stützelemente 23, zum Beispiel in Form von Pelotten ortsfest an dem Rückenteil 11 fixiert werden. Auch ist es denkbar, diese Stützelemente 23 durch zusätzliche Versteifungselemente zu verstärken,

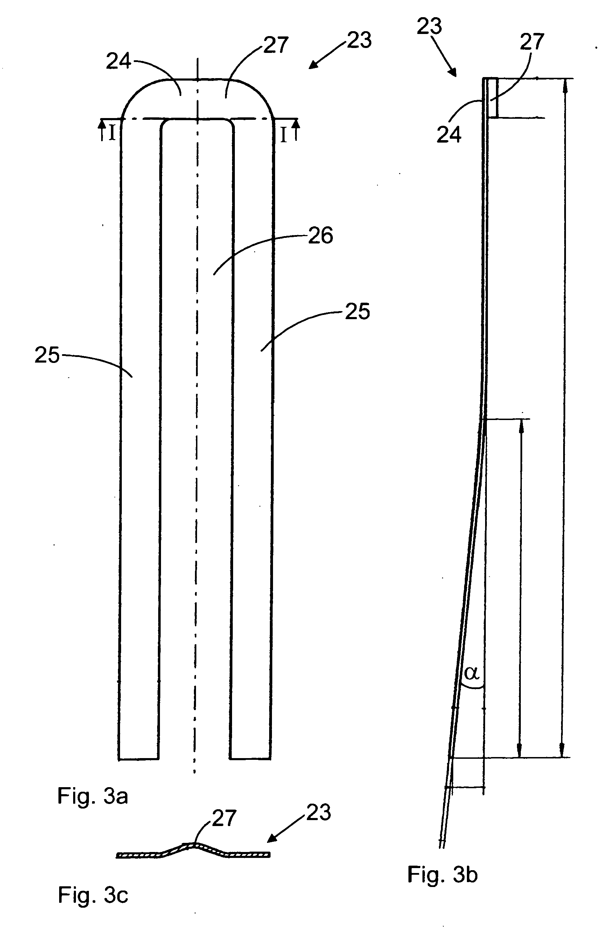

In der Fig. 3a ist ein bogenförmiges Stützelement 23 gezeigt, welches gleichzeitig als ein Kupplungslied 28 dient. Das bogenförmige Stützelement 23 ist an seinem kranialen Ende 24 geschlossen ausgebildet. Dagegen weist das Stützelement 23 zwei offene kaudale Enden 25 auf, die gleichzeitig zur Verbindung des Stützelementes 23 mit einem nicht dargestellten Miederstützelement 41 dienen. Zwischen den beiden stabförmigen kaudalen Enden 25 des Stützelementes 23 ist eine Ausnehmung 26, die ausreichend breit genug für die Wirbelsäule ist, vorgesehen.

In der Fig. 3b ist die Seitenansicht des bügelförmigen Stützelementes 23 dargestellt. Dabei ist deutlich zu erkennen, dass des Stützelement 23 ungefähr in der Mitte abgewinkelt (s. Winkel a) ist, wodurch das Stützelement 23 dem gesunden Verlauf der Brustwirbelsäule folgen soll. Weiter ist aus dieser Ansicht ein Bogen 27 am kranialen Ende 24 des Stützelementes 23 erkennbar. Auch dieser Bogen 27 dient dazu, einen Kontakt des Stützelementes 23 mit der Brustwirbelsäule zu vermeiden.

In der Fig. 3c ist der Querschnitt I aus Fig. 3a dargestellt. Auch in dieser Darstellung ist deutlich der Bogen 27 für die Wirbelsäule am kranlalen Ende 24 des Stützelementes 23 zu erkennen. Außerdem ist sichtbar, dass das Stützelement 23 extrem flach ausgestaltet ist, um einen hohen Tragekomfort für die BWS-Orthese zu gewährleisten. An dem flachen kaudalen Enden 26 können folglich problemlos die bereits genannten Versteifungselemente angebracht werden,

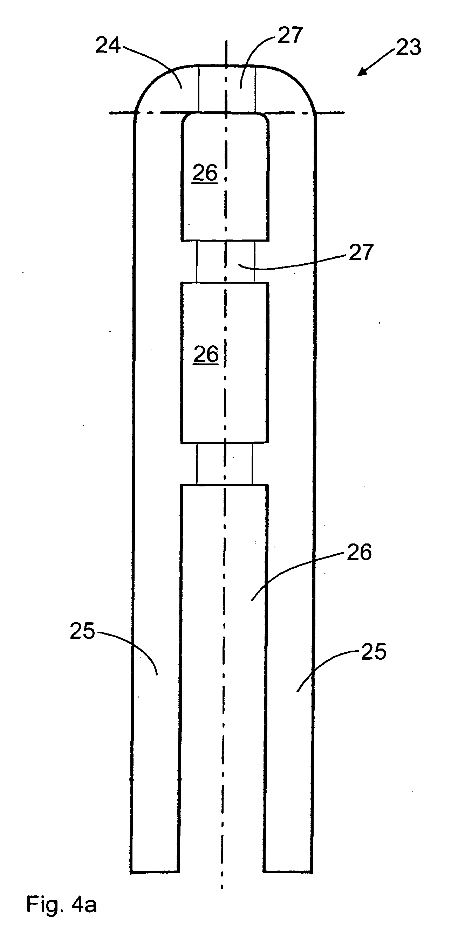

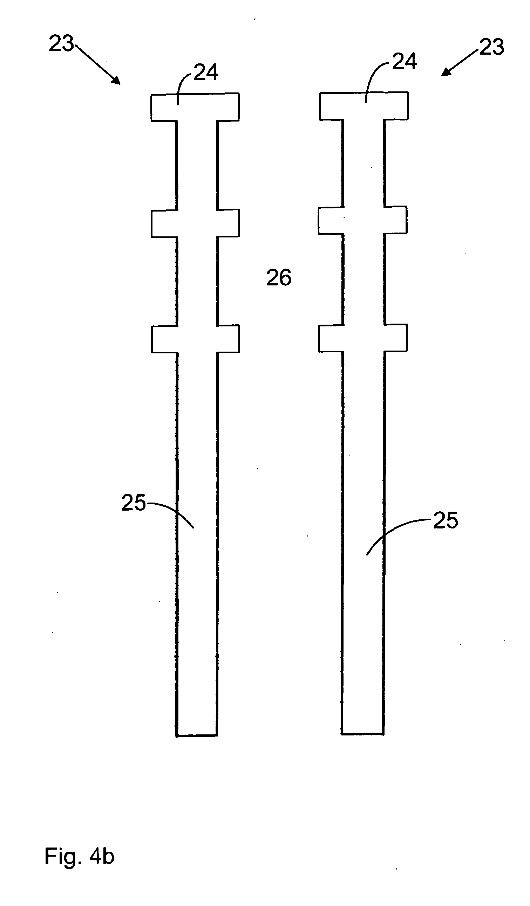

in den Figuren 4a und 4b sind weitere Stützelemente 23 in unterschiedlicher Ausgestaltung dargestellt. In der Fig. 4a kommt ebenfalls ein bügelförmiges Stützelement 23 zum Einsatz, wobei zusätzliche Querstreben die kaudalen Enden 26 des Stützelementes 23 verbinden, welche ebenfalls über Bögen 27 verzügen können. Somit wird eine erhöhte Stabilität des Stützelementes 23 erreicht. Außerdem ist die Befestigung des Stützelementes 23 an dem Rückenteil 11 bzw. den Taschen 21 erleichtert. Die unterste Querstrebe kann als Anschlagelement zum Gegenkupplungsglied 48 dienen.

In der Fig. 4b sind zwei gleiche, staubförmige Stützelement 23 in Draufsicht dargestellt. Auch dieses Stützelement 23 weist Querstreben zum Längsverlauf des Stütrelementes 23 auf. Ein derartiges Stützelement 23 kann links- und rechtsseitig von der Wirbelsäule angeordnet werden. Auch hierbei dient das kaudale Ende 25 des Stützetementes 23 zur Verbindung mit dem Miederstützelement 41. Zweckmäßigerweise werden auch bei einem Einsatz von zwei stabförmigen Stützelementen 23, wie aus Fig. 4b gezeigt, zwei entsprechend ausgestaltete Miederstützelemente 41 als Gegenkupplungsglieder 48 eingesetzt. Dabei können die zuvor genannten Querstreben als Anschläge dienen, damit die stabförmigen Stützelemente 23 nur bis zu einer vorgegebenen Tiefe mit dem Miederstützelement 41 zusammenwirken können.

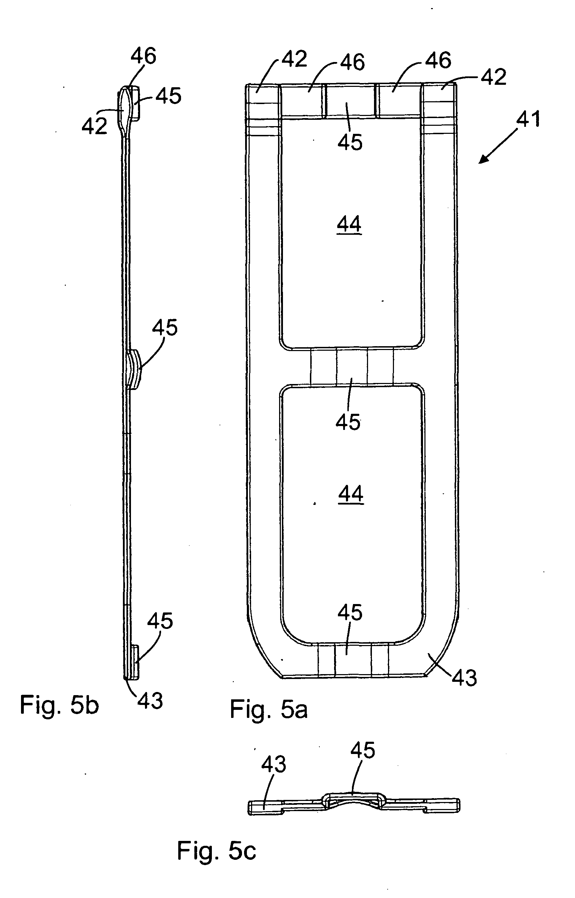

In der Fig. 5a ist ein rahmenförmiges Miederstützelement 41 dargestellt. Dieses Rahmentell verfügt über zwei Längsstäbe, die mit insgesamt drei Querstreben miteinander verbunden sind. An dem kranlalen Ende 42 des Miederstützelementes 41 ist ein Aufnahmebereich 46 vorgesehen, um ein entsprechendes Stützelement 23 fonnschlüssig aufnehmen zu können. Dabei besteht der Aufnahmebereich 48 aus einem Durchbruch, oder einer Bohrung, durch die die stebförmigen, flachen Enden 25 des Stützelementes 23 hindurchführbar sind. Auf diese Art und Welse ist das Kupplungsgiled 28, welches aus dem Stützelement 23 besteht, mit dem Gegenkupplungsglied 48, welches durch das Miederelement 41 verkörpert wird, teleskopartig Inelnanderschlebbar. Die somit verbundenen Stützelemente 23, 41 bieten eine ausreichende Stabilität der Wirbelsäule in der Sagittalebene.

In der Fig. 5b ist die Seitenansicht auf das rahmenförmlge Miederstützelement 41 aus Fig. 5a gezeigt. Dabei wird deutlich, dass die Querstreben ebenfalls bogenförmig 45 um die Wirbelsäule geführt werden. In dem rahmenförmigen Miederstützelement 41 sind ebenfalls Ausnehmungen 44 vorgesehen, um der Wirbelsäule ausreichend Platz zu verschaffen. In der Fig. 5c ist eine Rückansicht auf das Miederstützelement 41 aus Fig. 5a gezeigt.

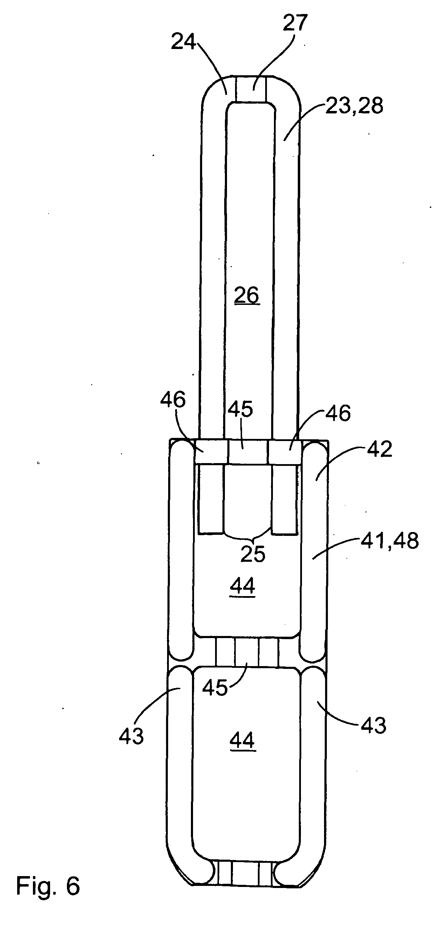

In der Fig. 6 ist das zusammengefügte Kupplungsglied 28 mit dem Gegenkupplungsglied 48 dargestellt. Dabei ist das Kupplungsglied 28 mit den kaudalen Enden 25 in Durchbrüche 46 des Gegenkupplungsgliedes 48 eingeführt. Damit Ist das Stützelement 23 bzw. das Kupplungsglied 28 mit dem Miederstützelement 41 bzw. dem Gegenkupplungeglied 48 formschlüssig verbunden. Auf diese Art und Weise wird die notwendige Stabilität der BWS-Orthese In der Sagittalebene erreicht. Zusätzlich können in den Gegenkupplungsglied 48 weltere Aufnahmenbereiche 46 bestehen, um die Stabilität der beiden miteinander verbundenen Stützelemente 23 und 41 zu erhöhen, Auch kann zusätzlich ein Adapterteil 30 eingesetzt werden, Im vorliegenden Beispiel aus Fig. 6 weist das rahmenförmige Miederstützelement 41 zusätzliche Versteifungselemente auf.

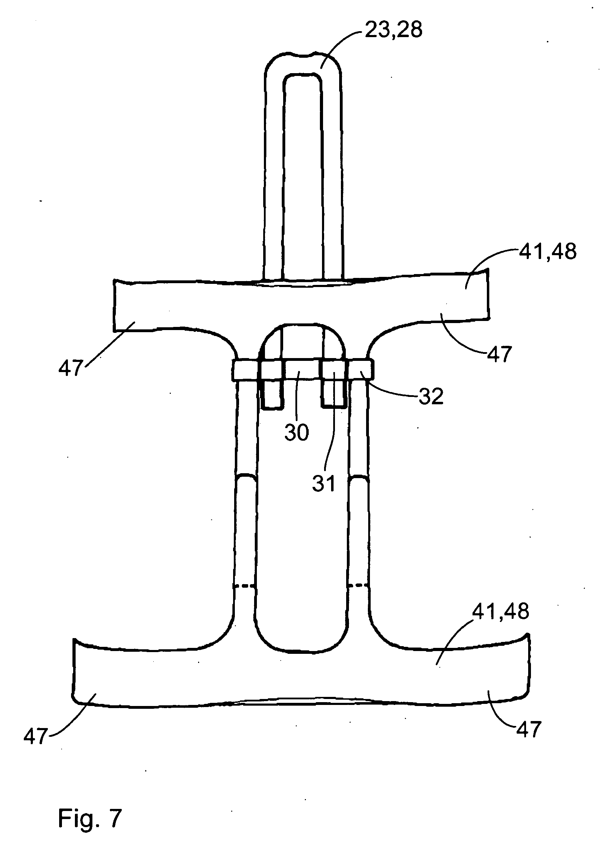

In der Fig. 7 ist weiteres Beispiel für ein Stützelement 23 und ein Miederstützelement 41 dargestellt. Dabei kommt zusätzlich ein Adapterteil 30 zum Einsatz, um die Stabilität der Verbindung zwischen dem Kupplungsglied 28 und dem Gegenkupplungsgiled 48 zu verbessem. Dieses Adapterteil 30 ist längsverschiebbar an den Stäben des rahmenförmigen Miederstützelementes 41 gehalten. Zusätzlich weist das Adapterteil 30 zwei unterschiedliche Aufnahmebereiche 31 und 32 auf. Dabei dient der Aufnahmebereich 31 zur formschlttasigen Aufnahme des Stützelementes 23, wo hingegen der Aufnahmebereich 32 zur forrnschlosslgon Aufnahme des Miederstützelementes 41 dient. Ferner sind an dem rahmenförmigen Miederstützelement 41 zwei bogenförmige Stützspangen 47 angeordnet. Das Miederstützelement 41 ist im vorliegenden Fall aus Fig. 7 selbst zweiteilig ausgestaltet, wobei eine Verbindung der beiden Teile durch die stabförmigen Elemente des Miederstützelementes 41 erfolgt. Durch die zweigeteilte Ausführung des Miederstützelementes 41 ist eine Größenverstellung möglich, wodurch die Orthese, insbesondere die Thorako-Lumbel-Orthene, optimal an den Körper des Patienten anpassbar ausgestaltet ist. Durch das zwelteilige Miederstützelement 41 mit den bogenförmigen Stützspangen 47 wird nicht nur eine Unterstützung der Wirbelsäule in der Sagittalebene sondern auch In der Frontalebene erreicht. Wie deutlich zu erkennen ist, sind die bogenförmigen Stützspangen 47 orthogonal zu den beiden Längsstäben des Miederstützelementes 41 angeordnet. Die bogenförmige Stützspange 47 kann mit den jeweiligen Längsstäben des Miederstützelementes 41 materieleinheitlich und einstückig verbunden sein. Die offenen Enden der Längsstäbe des Miederstützelementes 41 können über Rastmittel und Gegenrastmittel formschlüssig miteinander verbunden sein. Ebenfalls ist es denkbar, die zuvor genannten Enden über Klettverschlüsse oder Druckknöpfe oder dergleichen miteinander zu verbinden.

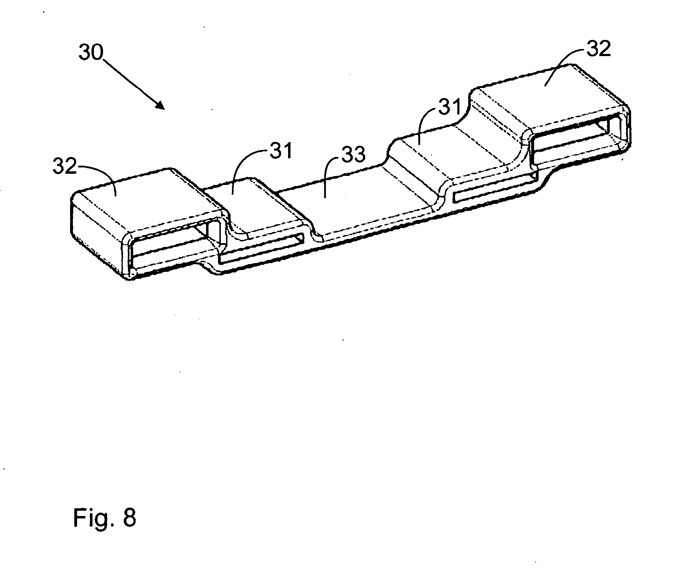

In der Fig. 8 ist in dreidimensionaler Ansicht das Adapterelement 30 dargestellt. Dabei sind jeweils links- und rechtsseitig die Aufnahmebereiche 32 für die stabförmigen Enden des Miederstützelementes 41 angeordnet. Diese bestehen im Wesentlichen aus einem Durchbruch, der rechteckförmig ausgestaltet Ist. An die Aufnahmebereiche 32 schließen sich nach innen die Aufnahmebereiche 31 für die offenen stabförmigen Enden des Stützelementes 23 an. Auch diese Aufnahmebereiche 31 weisen rechteckförmige Durchbrüche auf. Damit das Adapterteil 30 nicht auf die Wirbelsäule des Patienten drückt, ist zusätzlich eine mittige Aussparung 33 vorgesehen, die sich links- und rechtsseitig nach innen an die Aufnahmebereiche 31 anschließt. In der Fig. 7 wird die Verwendung des Adapterteils 30 gezeigt. Wie bereits erwähnt, ist dieses Adapterteils 30 auf den stabförmigen Enden des Miederstützelementes 41 höhenverstellbar befestigt. Auch die kaudalen Enden 25 des Stutzelementes 23 können veränderbar In den Aufnahmebereich 31 des Adapterteils 30 hineinragen. Alleine durch die Höhenverstellbarkeit des Adapterteils 30 kann die Stabilität der zusammengefügten Stützelemente 23 und 41 verändert werden. Das Adapterteil 30 kann selbst aus Kunststoff oder einem Metalltell, insbesondere einer Leichtmetalllegierung, bestehen.

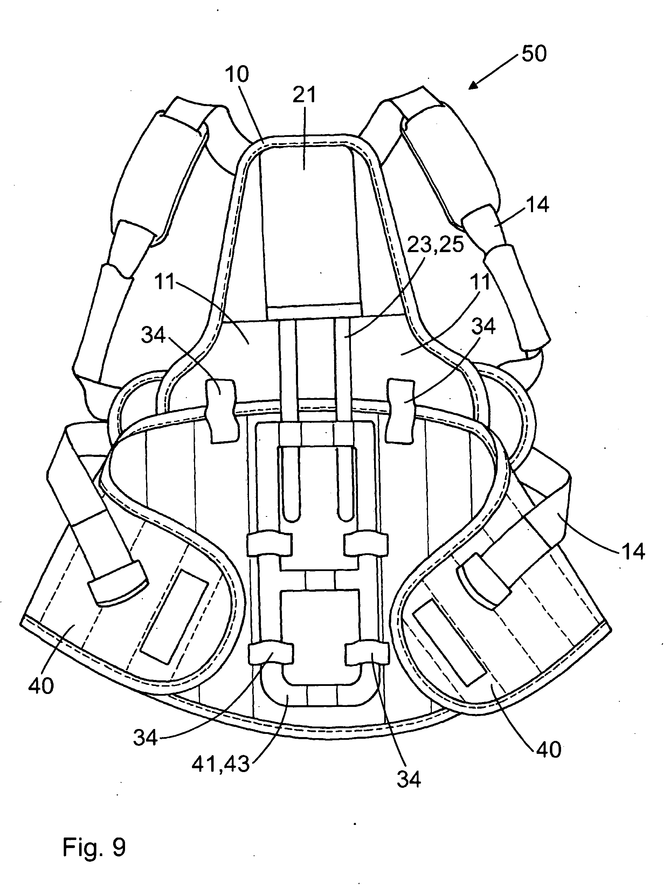

In der Fig. 9 ist eine nicht eigenständig beanspruchte Wirbelsäulen-Orthese 50 dargestellt, Diese Wirbdsäulen-Orthene 50 erhält man durch das Zusammenfügen der BWS-Orthesa 10 mit dem Lendenwirbeisäulen-Mieder 40. Zur Kopplung der BWS-Orthese 10 und des Lendenwirbelsäulen-Mieders 40 dient einerseits das Stützelement 23 und andererseits das Miederstützelement 41, die ein Kupplungsglied 28 und ein Gegenkupplungsglied 48 darstellen. Dabei ist das Stützelement 23 ortsfest in dem Rückentell 11 In der Tasche 21 angeordnet. Das Miederstützelement 41 kann über eine vergleichbare Tasche an der Innenseite des Lendenwirbelsäulen-Mieders fixiert werden. Ebenfalls ist es denkbar, wie im vorliegenden Fall, das rahmenförmige Miederstützelement 41 über Klettverschlusshalter 34 zu befestigen. Es sei an dieser Stelle erwähnt, dass sowohl das Stützelement 23 als auch das Miederstützelement 41 über andere Verbindungsmittel, wie zum Beispiel Druckknöpfe, Rast- und Gegenrastmittel fixierbar sind. Die in Fig. 9 dargestellte Wirbelsäulen-Orthese 50 unterstützt die Wirbelsäule ausschließlich in der Sagittalebene.

Die in der Fig. 9 dargestellte BWS-Orthese 10 ist über zusätzliche Verbindungselemente, wie zum Beispiel Klettverschlüsse, mit dem Lendenwirbelsäulen-Mieder 40 verbunden. Allerdings findet hierdurch keine Unterstützung der Wirbelsäule statt. Vielmehr ist somit eine entsprechende Anpassung der Wirbeisäulen-Orthese an die Körpergröße des Patienten möglich. Zu diesem Zweck ist auch das Kupplungsglied 28 verstellbar zum Gegenkupplungsglied 48 vorgesehen.

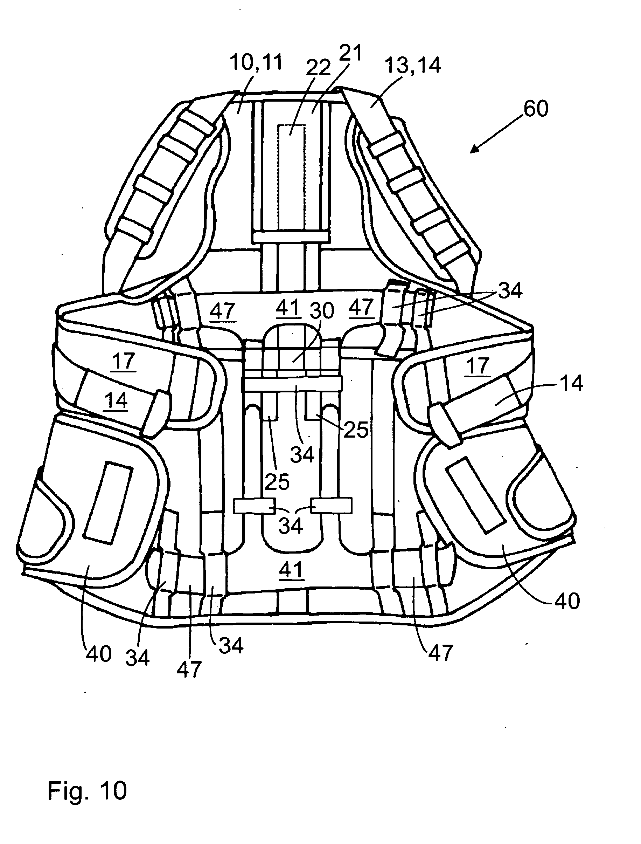

In der Fig. 10 ist eine Thorako-Lumbal-Orthese 60 als eine erfindungsgemäße Wirbelsäulenorthese dargestellt. Bei dieser Orthese 60 kommt die Kombination des Stützelementes 23 mit dem Miederstützelement 41 aus Fig. 7 zum Einsatz. Somit unterstützt die Thorako-Lumbal-Orthese 60 die aus der BWS-Orthese 10 und einem Tragriemensystem 17 mit dem Lendenwirbelsäulen-Mieder 40 besteht, die Wirbelsäule des Patienten in Sagittal- und Frontalebene. Dabei ist die BWS-Orthese 10 einerseits über das Kupplungsglied 28 und das Gegenkupplungsglied 48 miteinander verbunden. Zusätzlich können weitere Verbindungsmittel in Form von Klettverschlüssen, Druckknöpfen oder dergleichen vorgesehen sein. Das Gegenkupplungsglied 48 ist über Klettverschlusshalter 34 formschlüssig am Lendenwirbeisäulen-Mieder 40 gehalten. Das Miederstützelement 41 weist im vorliegenden Fall zwei bogenförmige Stützspangen 47 auf, die orthogonal zu den Stäben des Miederstützelementes 41 angeordnet sind.

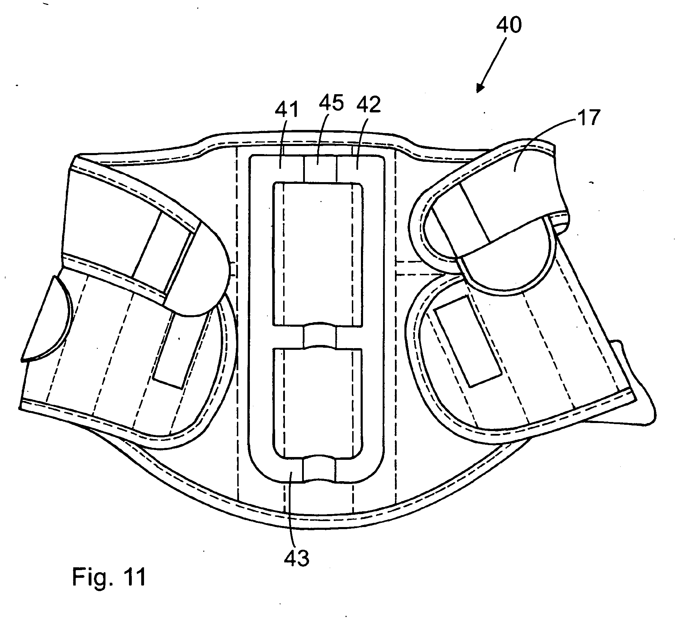

In der Fig. 11 ist ein nicht eigenständig beanspruchtes Lendenwirbelsäulen-Mieder 40 dargestellt. Dieses kann kranial mit einem Tragriemensystem 17 bzw. einem oberen Bauchmieder 17 verbunden sein. Dieses Tragriemensystem 17 ist wiederum kranial mit der BWS-Orthese 10 verbindbar. Das gezeigte Lendenwirbelsäulen-Mieder 40 Ist auch alleine einsetzbar, sofern der Patient nur im Lendenwirbelsäulenbereich Probleme hat. Hierzu kann das Miederstützelement 41 an der Innenseite des Lendenwirbalsäulen-Mieders 40, insbesondere formschlüssig, fixiert werden. Zur Anordnung des Miederstützelementes 41 können Taschen 21 oder Klettverschlusshalter 34 verwendet werden. Das Lendenwirbelsäulen-Mieder 40 kann kranial durch die BWS-Orthese 10 zu einer BWS/LWS-Orthese ergänzt werden.

In den Fig. 12a und 12b ist ein Gurthalter 16 mit einer Umlenkschnalle 35 dargestellt. Dabei weist diese Umlenkschnalle 35 zumindest zwei voneinander getrennt Durchziehlaschen 36 auf, die breit aber schmal ausgeführt sind; um gerade eben den unelastischen Gurt 14 bzw. das elastische Band 16 aufnehmen zu können. Wie aus der Fig. 12a erkennbar ist, sind die Ecken 38 der jeweiligen Durchziehlasche 36 abgerundet. Zusätzlich können diese Ecken 38 verstärkt ausgestaltet sein, um Risse oder Schnitte in den Gurten bzw. Bändern zu den Scheuerstellen zu vermeiden. Zusätzlich ist an der Umlenkschnalle 35 eine Annähfahne 37 angeordnet, die ungefähr die Breite des unelastischen Gurtes 14 aufweist. Diese Annähfahne 37 dient zum zusätzlichen Annähen des elastischen Bandes 16 an der Umlenksohnalle 35, um ein "Zusammenschieben" des elastischen Bandes 16 an den Gurthalter 15 zu vermeiden.

Die Fig. 12b stellt den Schnitt **II** durch die Fig. 2a dar. Dabei ist deutlich die Trennung der beiden Durchziehlaschen 36 zu erkennen. Auch ist der keilförmige Verlauf der

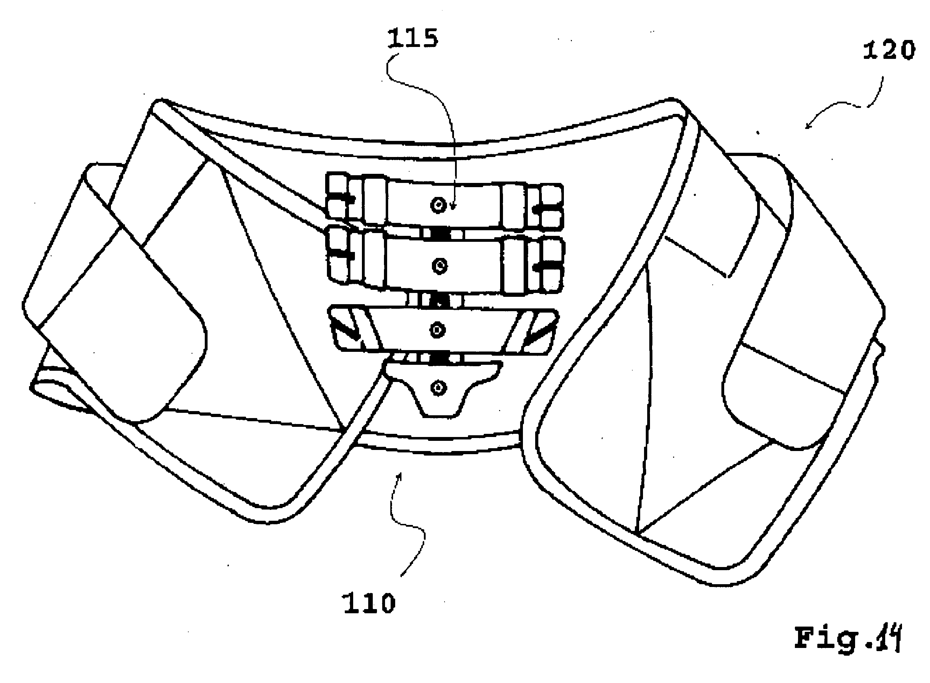

Die Figuren 14, 17, 21, 22, 24, 26 und 28 zeigen sieben unterschiedliche Orthesen, die aus dem Baukastensystem konfigurierbar sind. Die übrigen Figuren zeigen einzelne Elemente des Baukastens, die noch keine tragbare Orthese darstellen.

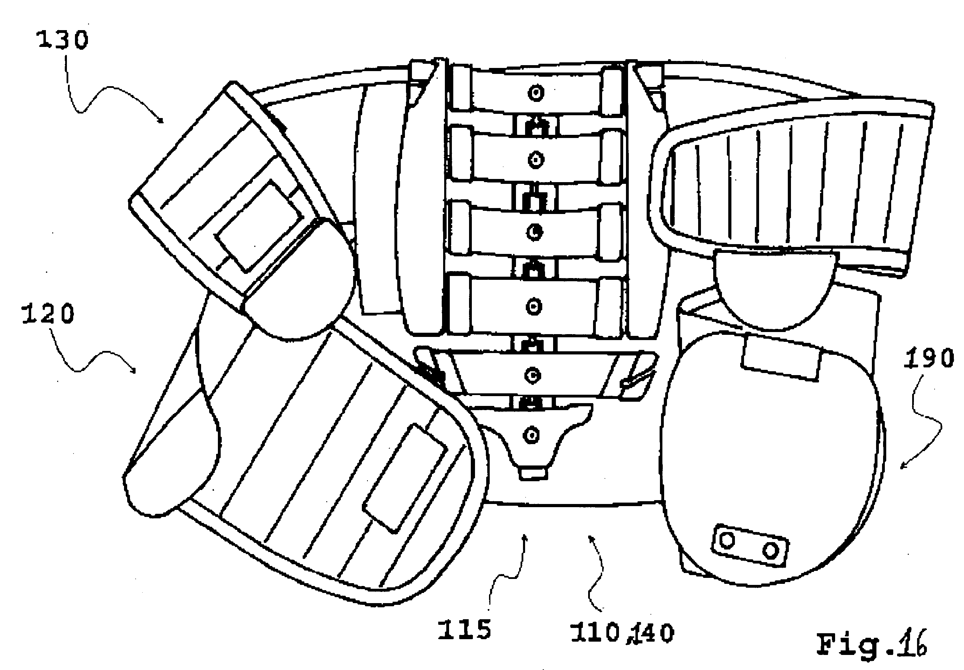

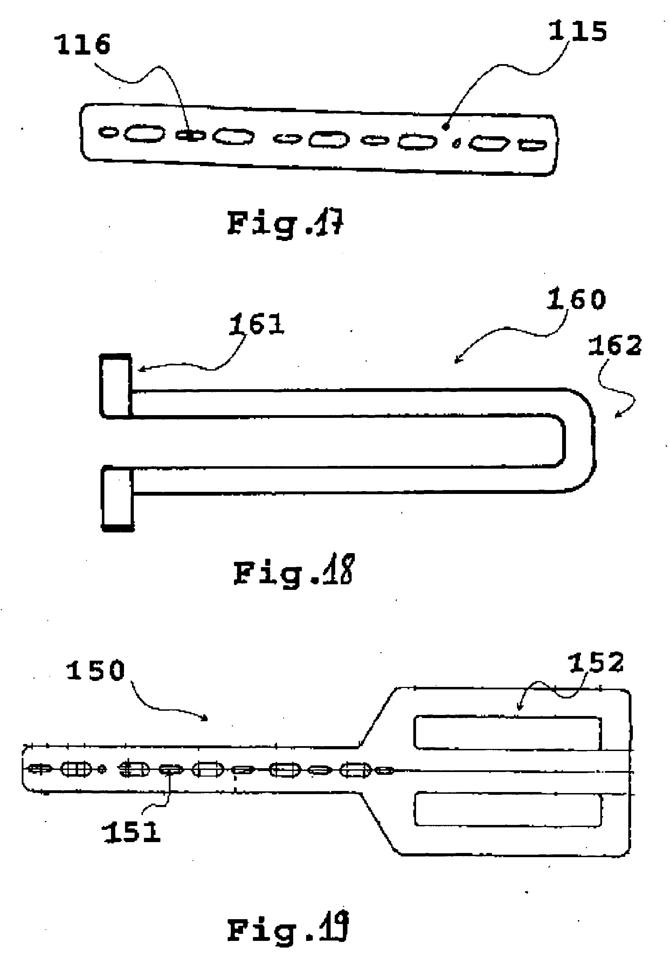

Ein Wesentliches Basiselement des zweiten grundlegenden Baukastensystems ist die in Fig. 13 dargestellte Basispelotte in Gestalt einer Gliederpelotte 110. Diese besteht aus vier miteinander gelenkig verbundenen Gliedern 111, die je einem Wirbel zugeordnet sind. Das unterste Glied ist dem ersten Kreuzwirbel (S1) zugeordnet, die drei weiteren Glieder den dritten bis zum fünften Lendenwirbel (L3-L5). Die Glieder 111 sind brückenartig ausgeführt und überbrücken "ihren" Wirbel transversal und liegen mit Auflageflächen 112 jeweils beiderseits des Wirbels auf dem Rücken auf. Lateral weisen die den Lendenwirbeln zugeordneten Glieder 111 nicht auf dem Rücken aufliegende Steckenden 113 auf, auf die später beschriebene Stützschienen 142 der Lumbalerweiterung 140 aufgesetzt werden können. Die Glieder 111 der Gliederpelotte 110 lassen sich mit Hilfe von Gelenken 114 gelenkig dergestalt verbinden, dass die einzelnen Glieder 111 sich in allen drei Ebenen (Frontal-, Sagittal- und Transversalebene) zueinander beweglich sind. Diese Beweglichkeit wird eingeschränkt durch wahlweises Festsetzen eines in Fig. 16 dargestellten Fixierelements 115 oder eines in Fig. 19 dargestellten Verbindungselements 150 posterior auf die Gliederpelotte 110. Hierzu besitzen die einzelnen Glieder 111 der Gliederpelotte 110 auf ihrer Rückseite Druckknöpfe, die in Aussparungen 151 des Fixierelementes 115 bzw. des Verbindungselements 150 einrasten. Die einzelnen Glieder 111 der Gliederpelotte 110 werden dadurch in Sagittalebene unbeweglich untereinander fixiert, so dass im unteren Lumbalbereich lediglich eine gewisse Bewegungsfreiheit in Frontal- und Transversalebene erhalten bleibt.

Die mit dem Fixierelement 115 in ihrer sagittalen Beweglichkeit eingeschränkte Gliederpelotte 110 kann dorsal in einem unteren Bauchmieder 120 festgeklettet werden. Auf diese Weise erhält man die in Fig. 14 dargestellte Stabilisierungsorthese, welche den Lendenwirbelbereich stützt und aufrichtet. Hierzu wird das untere Bauchmieder 120 entsprechend um den Bauch geschlungen und zugeklettet. Diese Basisorthese stützt und entlastet den unteren Lumbalbereich.

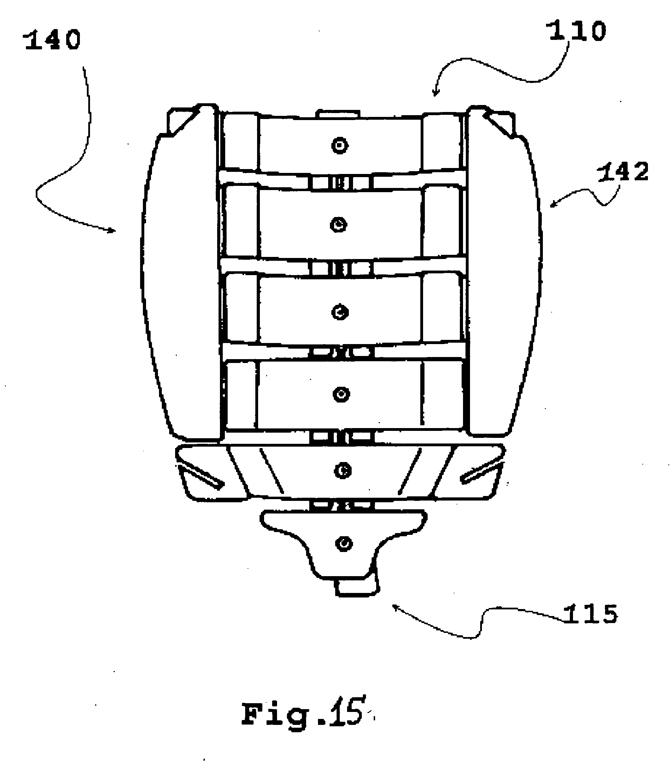

Wenn die gesamte Lendenwirbelsäule delordosiert werden soll, kann die Gliederpelotte 110 um eine in Fig. 15 dargestellte Lumbalerweiterung 140 erweitert werden. Die Lumbalerweiterung 140 umfasst zwei weitere Glieder 141, die in die bestehende Gliederpelotte 110 eingegliedert werden, so dass die erweiterte Gliederpelotte 110 insgesamt sechs Glieder 111, 141 umfasst, die den gesamten Lumbalbereich (L1 - L5), sowie den ersten Kreuzwirbel (S1) überdecken. Zusätzlich umfasst die Lumbalerweiterung 140 zwei Stützschienen 142, die lateral auf die Steckenden 113 zumindest der vier Glieder 111, 141 der erweiterten Gliederpelotte 110, 140 aufsetzbar sind, welche die ersten vier Lendenwirbel überbrücken. Die aufgesetzten Stützschienen 142 führen zu einer Versteifung des Pelottenverbandes in allen drei Ebenen, so dass sich lediglich die Glieder des fünften Lendenwirbels, sowie des ersten Kreuzwirbels noch frei bewegen lassen. Der Patient kann sich im durch die Stützschienen 142 versteiften Bereich nur noch eingeschränkt innerhalb der Frontalebene bewegen, die sagittale Beweglichkeit ist vollständig blockiert.

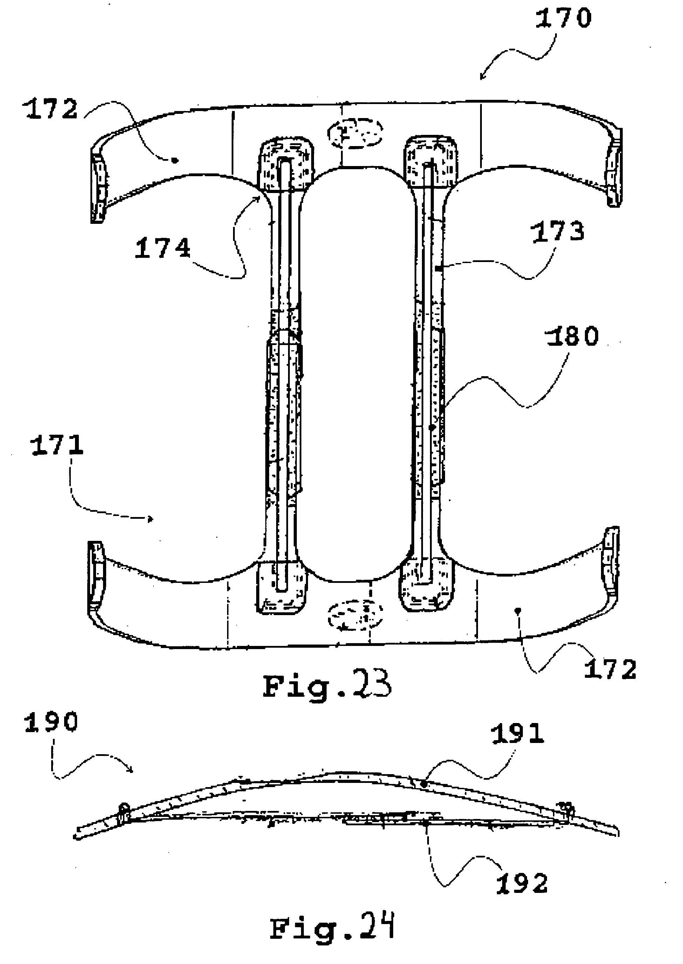

Die in Fig. 15 dargestellte, um die Lumbalerweiterung 140 erweiterte Gliederpelotte 110 kann dorsal in das untere Bauchmieder 120 eingeklettet werden. Da dieses nicht den gesamten Lendenwirbelbereich überstreicht, wird es kranial um ein oberes Bauchmieder 130 ergänzt. Oberes und unteres Bauchmieder 120, 130 sind durch Klettverschlüsse aneinander festsetzbar. Der Überlappungsbereich zwischen unterem und oberen Bauchmieder 120, 130 ist frei wählbar, so dass sich der Miederverband an die Körpergröße des Patienten anpassen lässt. Zur Steigerung des intraabdominalen Drucks kann ventral in dem unteren Bauchmieder 120 eine Bauchpelotte 190 eingeklettet werden. Die Bauchpelotte 190 ist in Fig. 24 dargestellt und wird später noch näher erläutert. Bei der aus unterem Bauchmieder 120, oberen Bauchmieder 130, Gliederpelotte 110, Lumbalerweiterung 140, Fixierelement 115 und Bauchpelotte 190 bestehende Orthese in Fig. 16 handelt es sich um eine Lumbalorthese, die den gesamten Lendenwirbelbereich entlordosiert.

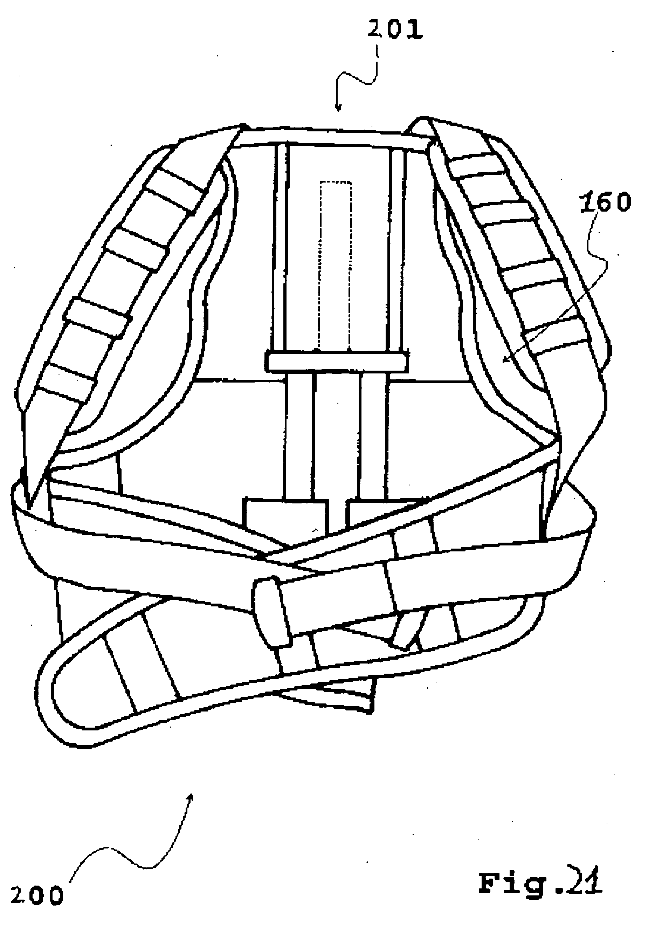

Wenn die Wirbelsäule darüber hinaus auch im Brustwirbelbereich gestützt werden soll, ist die Gliederpelotte 110 mit Hilfe des in Fig. 18 dargestellten Stützelements 160 erweiterbar. Das Stützelement 160 ist im Wesentlichen U-förmig und erstreckt sich angelegt mit seinen Schenkeln entlang der Wirbelsäule im Brustwirbelbereich. An seinem kaudalen Ende weist das Stützelement 160 an jedem Schenkel eine Klettklammer 161 auf, mit Hilfe deren das Stützelement 160 wahlweise an dem in Fig. 19 dargestellten Verbindungselement 150 oder dem später beschriebenen und in Fig. 23 dargestellten Überbrückungsrahmen 170 festklettbar ist. Mit seinem kranialen Ende 162 wird das Stützelement 160 in eine hierfür eigens vorgesehene Tasche 201 eingeschoben, die sich dorsal an einem rucksackartigen Brustwirbelsäulenmieder 200 befindet. Das Brustwirbelsäulenmieder 200 ist in Fig. 21 mit eingeschobenem Stützelement 160 dargestellt. Das Stützelement 160 wird in der Tasche 201 des Brustwirbelsäulenmieders 200 festgeklettet. Diese in Fig. 21 dargestellte Orthese ist einzeln als Thoraxorthese tragbar.

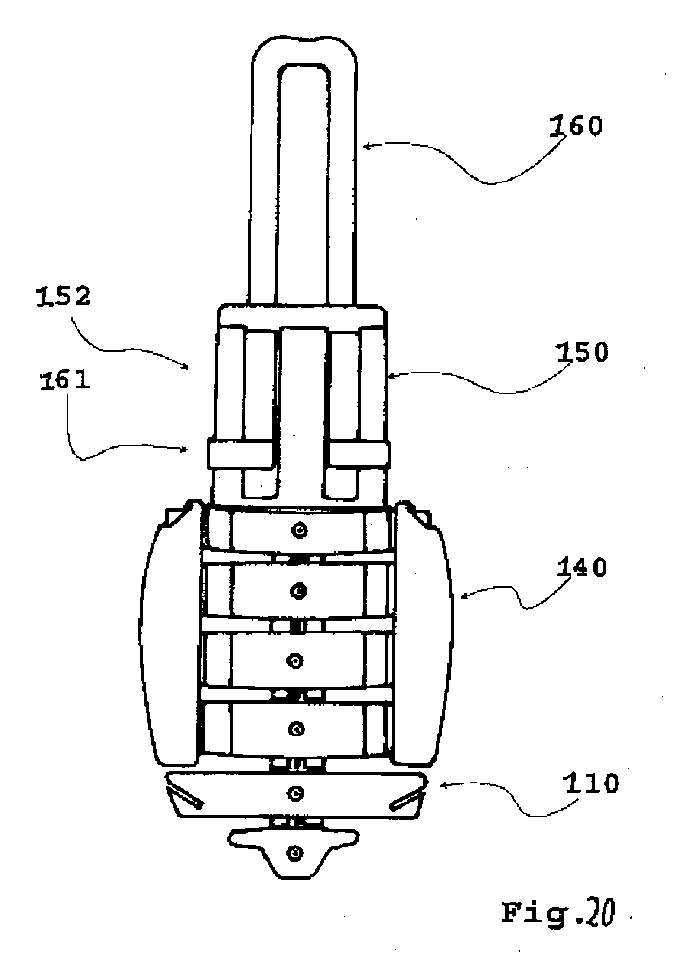

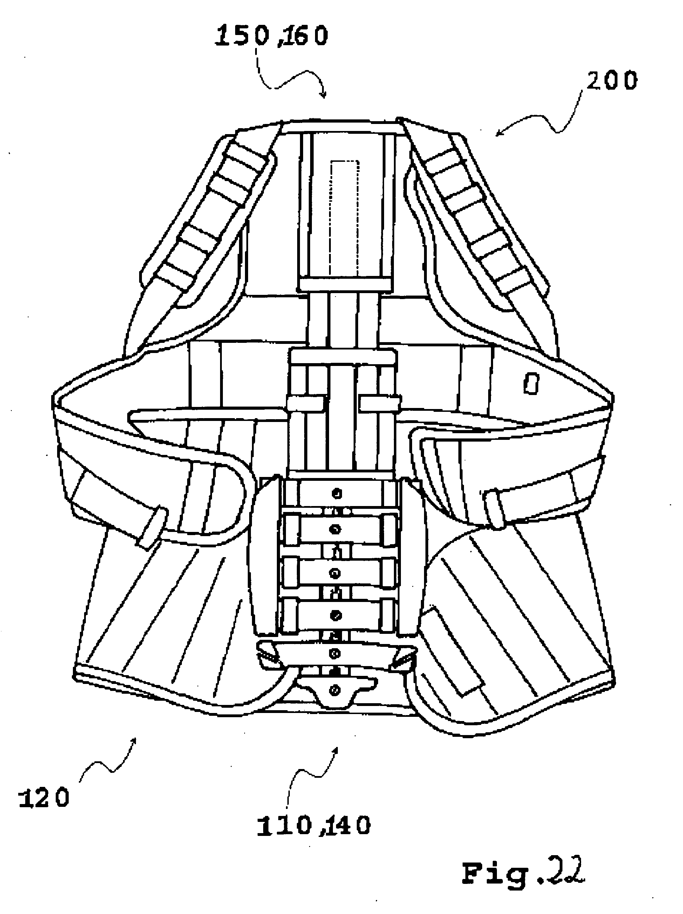

Mit Hilfe des Stützelements 160 und dem Brustwirbelsäulenmieder 200 lässt sich die in Fig. 16 dargestellte Lumbalorthese in die in Fig. 22 dargestellte Wirbelsäulenorthese aufbauen, welche die gesamte Wirbelsäule in der Sagittalebene stabilisiert und aufrichtet. Hierzu wird zunächst das in Fig. 17 dargestellte Fixierelement 115 durch das in Fig. 19 dargestellte Verbindungselement 150 ersetzt. Dieses weist an seinem kranialen Ende eine gabelartige Aufnahme 152 auf, in der das Stützelement 160 mit seinen Klettklammern 161 festklettbar ist. Auf diese Weise entsteht die in Fig. 20 dargestellte Stützstruktur. Diese kann an die Körpergröße des Patienten dadurch angepasst werden, dass die Klettklammern 161 innerhalb der gabelartigen Aufnahme 152 des Verbindungselements 150 auf die notwendige Länge eingeschoben wird.

Schließlich wird das Brustwirbelsäulenmieder 200 anstelle des oberen Bauchmieders 130 kranial am unteren Bauchmieder 120 festgeklettet und die in Fig. 20 dargestellte Stützstruktur in das aus unterem Bauchmieder 120 und Brustwirbelsäulenmieder 110 bestehende Gesamtmieder dorsal eingesetzt. Auf diese Weise entsteht die in Fig. 22 dargestellte Wirbelsäulenorthese, welche den betroffenen Wirbelsäulenbereich vom Beckenkamm bis einschließlich zum Thorax überbrückt, dabei die Wirbelkörper entlastet, den gesamten Wirbelsäulenbereich in der Sagittalebene stabilisiert und aufrichtet. Im Lendenwirbelsäulenbereich sorgen die um die Lumbalerweiterung 140 erweiterte Gliederpelotte 110 und das stabilisierende Verbindungselement 150 für eine Delordosierung. Aufgrund des im oberen Bereich eingesetzten Stützelements 160 wird eine Aufrichtung der Brustwirbelsäule erzielt und die Hyperkyphose reduziert. Ebenso die Lendenlordose wird durch die in Fig. 22 dargestellte Wirbelsäulenorthese deutlich vermindert, die Brustwirbelsäule wird dadurch aufgerichtet. Aufgrund des modularen Charakters kann die Orthese jederzeit bei eingetretener Linderung in bestimmten Wirbelsäulenbereichen durch Entnahme der jeweiligen Stützelemente zurückgebaut werden, so dass eine therapeutisch korrekte Abschulung gestattet ist. Sollten sich die Beschwerden später erneut verstärken, kann jederzeit zu der vollständigen Wirbelsäulenorthese zurückgekehrt werden.

Insbesondere durch Osteoporose bedingte Leiden erfordern eine Aufrichtung und Entlastung der Wirbelsäule in Sagittal- und Frontalebene. Hierzu enthält das Baukastensystem einen in Fig. 23 dargestellten Überbrückungsrahmen 170, der anstelle der lediglich in Sagittalebene stützende Gliederpelotte 110 in einen Verband aus unterem und oberen Bauchmieder 120, 130 einklettbar ist, so dass die in Fig. 24 dargestellte Überbrückungsorthese entsteht. Der Überbrückungsrahmen 70 besteht im Wesentlichen aus zwei II-förmigen Halbelementen 171. An ihren kaudalen, bzw. kranialen Ende weisen die Halbelemente 171 einen bogenförmigen Träger 172 auf, der den unteren Thorax bzw. das Becken stützend umschließt. Von den Trägern 172 aus laufen jeweils zwei Verbindungsstreben 173 entlang der Wirbelsäule in Richtung des anderen Halbelements 172. Die Verbindungsstreben 173 sind mit Klettverschlüssen ausgerüstet und können somit aneinander befestigt werden, so dass der Abstand der Träger 172 an die Körpergröße des Patienten anpassbar ist. Darüber hinaus können auf den Verbindungsstreben 173 zusätzliche Versteifungsstäbe 180 befestigt werden, in dem diese in entsprechende Taschen 174 an den Trägern 172 eingesteckt werden. Durch Einstecken der Versteifungsstäbe 180 kann die Steifigkeit des Überbrückungsrahmens 170 an den jeweiligen Genesungsfortschritten des Patienten angepasst werden.

Zur Feineinstellung der Steifigkeit können unterschiedliche Werkstoffe wie beispielsweise Kunststoff oder Metall für die Versteifungsstäbe 180 vorgesehen werden.

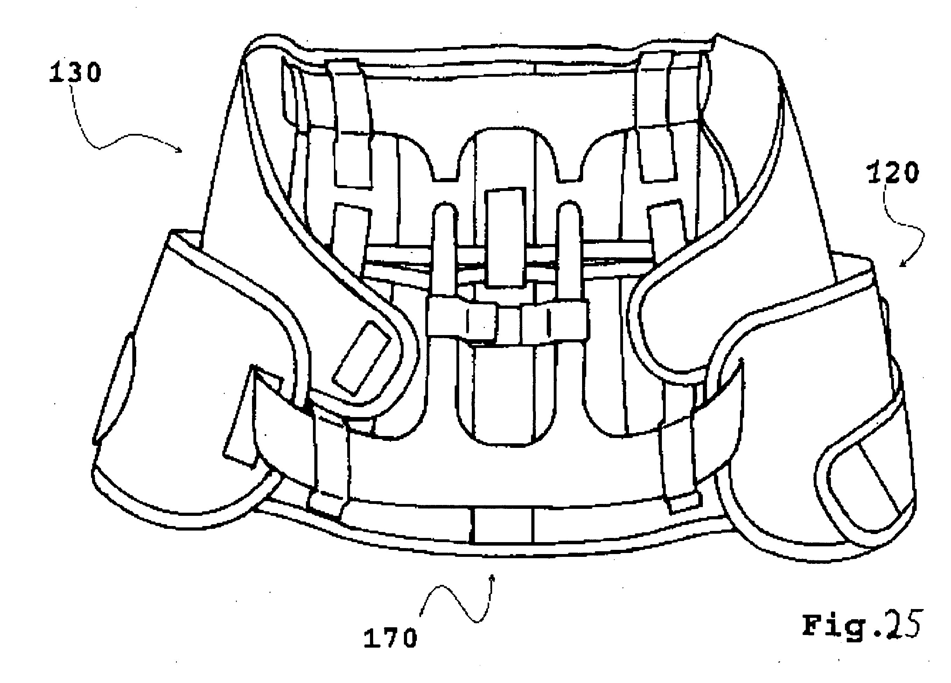

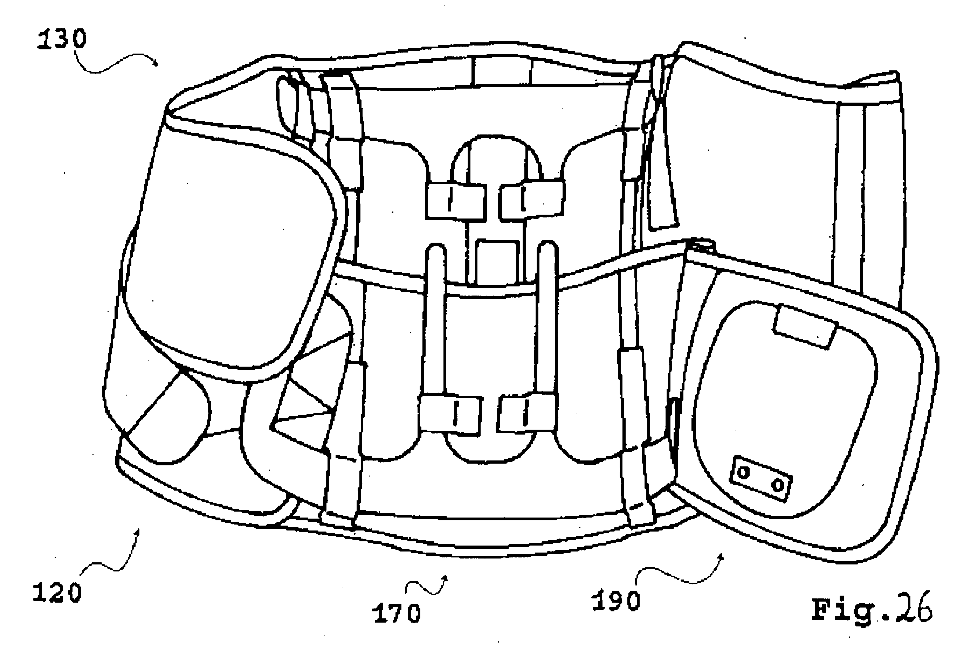

Um die in Fig. 25 dargestellte Überbrückungsorthese in eine in Fig. 26 dargestellte Flexionsorthese aufzurüsten, wird in das untere Bauchmieder 120 ventral eine in Fig. 24 in Seitenansicht dargestellte Bauchpelotte 190 eingeklettet. Die Bauchpelotte 190 umfasst eine Druckplatte 191, deren konkave Krümmung mit Hilfe zweier Laschen eines Klettbandes 192 eingestellt wird. Durch entsprechendes Vorspannen des Klettbandes 192 wird das konvexe Profil der Druckplatte 191 auf das gewünschte Maß angepasst. Die gesamte Bauchpelotte 190 kann in das untere Bauchmieder 120 über das Klettband 192 ventral eingeklettet werden.

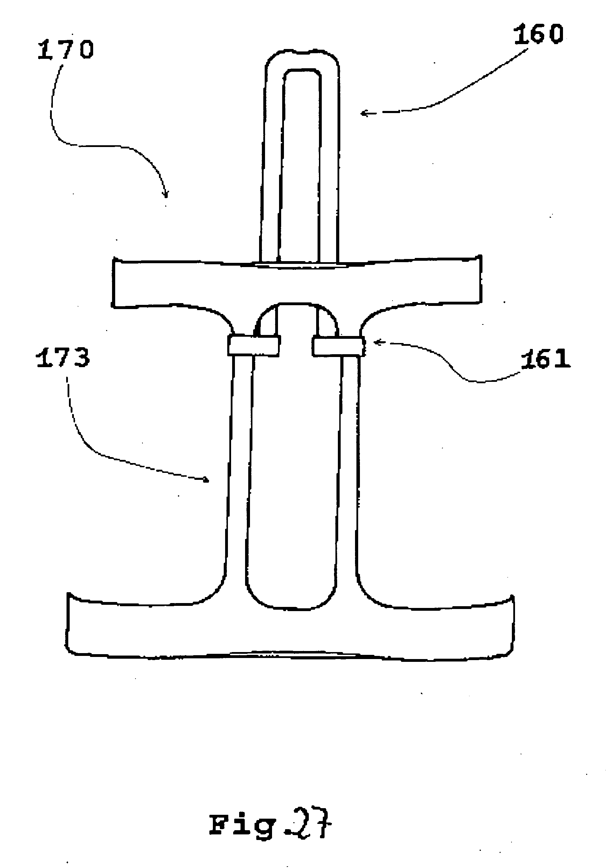

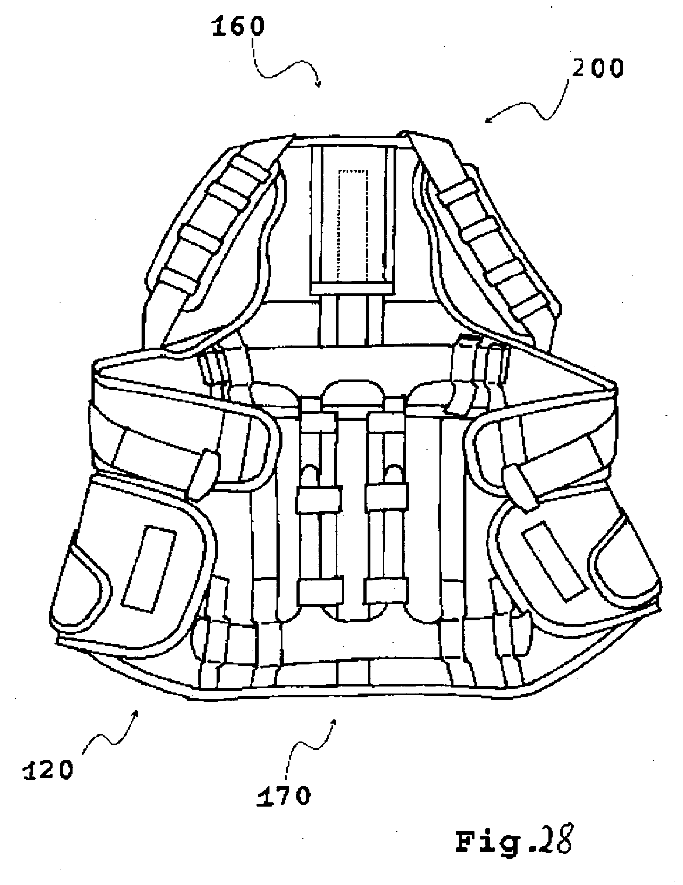

Mit Hilfe des U-förmigen Stützelements 160 und dem Brustwirbelsäulenmieder 200 kann die in Fig. 25 dargestellte Überbrückungsorthese in eine in Fig. 28 abgebildete Osteoporoseorthese aufgerüstet werden, die den gesamten Wirbelsäulenbereich vom Beckenkamm bis einschließlich des Thorax in der Sagittal- und Frontalebene stabilisiert und aufrichtet. Hierzu wird das aus Fig. 18 bekannte Stützelement 160 mit seinen Klettklammern 61 auf die Verbindungsstreben 173 des Rahmens 130 festgesetzt. Eine Höhenverstellung ist durch freie Wahl des Anklettungspunktes problemlos möglich. Der so entstehende, in Fig. 27 dargestellte Verband wird kaudal in das untere Bauchmieder 120 und kranial in dem Brustwirbelsäulenmieder 200 eingeklettet, das ebenfalls an dem unteren Bauchmieder 120 befestigt wird. Zudem wird das Stützelement 160 mit seinem kranialen Ende 162 in die dafür vorgesehene Tasche 201 des Brustwirbelsäulenmieders 200 eingeschoben, so dass schließlich die in Fig. 28 dargestellte Osteoporoseorthese entsteht.

Diese sorgt dank des höhenverstellbaren Überbrückungsrahmens 170 für eine deutliche Delordosierung, die der kompensatorischen Hyperlordose entgegenwirkt. Durch das im oberen Bereich eingesetzte Stützelement 160 wird eine Aufrichtung der Brustwirbelsäule erzielt, die Hyperkyphose dabei deutlich reduziert. Zusätzlich wird die Lendenlordose deutlich vermindert, die Brustwirbelsäule aufgerichtet. Im Gegensatz Lendenlordose deutlich vermindert, die Brustwirbelsäule aufgerichtet. Im Gegensatz zu anderen Ostaoporosnorthesen ist durch den modularen Charakter des aus dem Baukastensystem konfigurierten Orthese eine spätere Reduktion auf den jeweils betroffenen schmerzhaft erkrankten Bereich möglich. Sollte sich beispielsweise eine Linderung im Lumbalbereich einstellen, wird das untere Bauchmleder 120 zusammen mit dem Stützrahmen 170 entfernt und fortan lediglich das Brustwirbelsäulenmieder 100 mit eingestecktem Stützelement 160 getragen, so wie in Fig. 19 dargestellt. Darüber hinaus ist eine therapeutische korrekte Abschulung über verschiedene Therapiestufen möglich durch Einsatz von erst starren, dann flexiblen Versteifungsstäben 180 am Überbrückungsrahmen 170. Sollten sich die Beschwerden später erneut verstärken, ist jederzeit eine Rückkehr zur vollständigen Wirbelsäulenorthese möglich.

### Bezugszeichenliste

- 10: BWS-Orthese
- 11: Rückenteil
- 12: streifenförmige Bahn
- 13: Schultergurtsystem
- 14: unelastischer Gurt
- 15: Gurthalter
- 16: elastisches Band
- 17: Trageriemensystem
- 18: Riemen
- 19: stationäres Riemenende
- 20: offenes Riemenende z. B. mit Klettverschlussteilen
- 21: Tasche
- 22: Klettverschluss für Tasche
- 23: Stützelement
- 24: kraniales Ende des Stützelementes
- 25: kaudales Ende des Stützelementes
- 26: Ausnehmung im Stützelement
- 27: Bogen (um Wirbelsäule) im Stützelement
- 28: Kupplungsglied
- 29: Befestigungslappen
- 30: Adapterteil
- 31: Aufnahmebereich für 23
- 32: Aufnahmebereich für 41
- 33: Aussparung im Adapterteil
- 34: Klettverschlusshalter
- 35: Umlenkschnalle
- 36: Durchziehlasche
- 37: Annähfahne
- 38: abgerundete Ecke
- 40: Lendenwirbeisäulen-Mieder
- 41: Miederstützelement
- 42: kranlales Ende des Miederstützelementes
- 43: kaudales Ende des Miederstützelementes
- 44: Ausnehmung im Miederstützelement
- 45: Bogen (um Wirbelsäule) im Miederstützelement
- 46: Aufnahmebereich bzw. Durchbruch
- 47: bogenförmige Stützepange
- 48: Gegenkupplungsglied

- 50: Wirbelsäulen-Orthese

- 60: Thorako-Lumbal-Orthese

- 110: Gliederpelotte
- 111: gelenkig verbundene Glieder
- 112: Auflagefläche
- 113: Aufliegende Steckenden
- 114: Gelenke
- 115: Fixierelement

- 120: unteres Bauchmieder
- 130: oberes Bauchmieder
- 140: Lumbalerweiterung
- 141: Glieder
- 142: Stützschiene
- 150: Verbindungselement
- 151: Aussparung
- 152: gabelartige Aufnahme
- 160: Stützelement
- 161: Klettklammer
- 162: kraniales Ende
- 170: Überbrückungsrahmen
- 171: Halbelemente
- 172: bogenförmige Träger
- 173: Verbindungsstreben
- 174: Taschen
- 180: Versteifungsstäbe

- 190: Bauchpelotte
- 191: Druckplatte
- 192: Klettband
- 200: Brustwirbeisäulenmieder
- 201: Tasche

- L1: Lendenwirbel 1
- L2: Lendenwirbel 2
- L3: Lendenwirbel 3
- L4: Lendenwirbel 4
- L5: Lendenwirbel 5
- S1: erster Kreuzwirbel

## Patentansprüche

1. Baukastensystem zum Konfigurieren einer Wirbelsäulenorthese aus einem oder aus mehreren Elementen des Baukastensystems, welches die folgenden Elemente enthält:
a1) ein unteres Bauchmieder (40,120),
b1) ein kranial am unteren Bauchmieder (40,120) festsetzbares oberes Bauchmieder (17,130),
c1) eine dorsal in dem unteren Bauchmieder (40,120) festsetzbares rahmenförmiges Miederstützelement (41), welches entlang der Lendenwirbelsäule anordbar ist und die Wirbelsäule unter Einschränkung der sagittalen Beweglichkeit stützt,
e1) ein kranial am unteren Bauchmieder (40,120) festsetzbares Brustwirbelsäulenmieder (10,200),
g1) wenigstens eine posterior wahlweise in einen Verband aus unterem Bauchmieder (40,120) und oberem Bauchmieder (17,130) oder in einen Verband aus unterem Bauchmieder (40,120) und Brustwirbelsäulenmieder (10,200) einsetzbare bogenförmige Stützspange (47), die an dem rahmenförmigen Miederstützelement (41) anordbar ist und die zur Entlordosierung und Einschränkung sagittalen und frontalen Beweglichkeit im Lendenwirbelsäulenbereich dient,
h1) zumindest ein kranial im Brustwirbelsäulenmieder (10,200) und kaudal wahlweise am rahmenförmigen Miederstützelement (41) festsetzbares Stützelement (23,160), welches sich seitwärts entlang der Wirbelsäule erstrecken kann, um deren Aufrichtung und Entlastung in der Sagittalebene zu erzielen,
i1) und eine ventral in dem unteren Bauchmieder (40,120) festsetzbare Bauchpelotte (190) zur Entlordosierung der Lendenwirbelsäule und Steigerung des intraabdominalen Druckes.

2. Baukastensystem nach Anspruch 1,
**dadurch gekennzeichnet,**
**dass** das Stützelement (23) über ein Kupplungsglied (28) und das Miederstützelement (41) über ein Gegenkupplungsglied (48) aufweist, die insbesondere über einen Formschluss zusammenfügbar sind.

3. Baukastensystem nach Anspruch 1 oder 2,
**dadurch gekennzeichnet,**
**dass** das Brustwirbelsäulenmieder (10,200) ein Schultergurtsystem (13), womit ein insbesondere ein großflächiges Rückenteil (11) rucksackartig gehalten ist, sowie ein ventrales Trageriemensystem (17), welches ebenfalls mit dem Rückenteil(11) verbunden ist, aufweist.

4. Baukastensystem nach Anspruch 3,
**dadurch gekennzeichnet,**
**dass** das Schultergurtsystem (13) unelastische Gurte (14) enthält, die insbesondere ein Kunststofftextil aufweisen.

5. Baukastensystem nach Anspruch 3 oder 4,
**dadurch gekennzeichnet,**
**dass** das Schultergurtsystem (13) über Gurthalter (15) am Rückenteil (11) angeordnet ist, wobei die Gurthalter (15), insbesondere über elastische Bänder (16), unlösbar an dem Rückenteil (11) befestigt sind.

6. Baukastensystem nach einem der Ansprüche 1 bis 5,
**dadurch gekennzeichnet,**
**dass** das Trageriemensystem (17) bzw. das obere Bauchmieder (130) über Befestigungslappen (29) an dem Rückenteil (11) reversibel befestigbar ist.

7. Baukastensystem nach Anspruch 6,
**dadurch gekennzeichnet,**
**dass** die Befestigungslappen (29) doppelwandig ausgestaltet sind, wobei die Innenseiten mit Klettverschlüssen versehen sind, die mit entsprechend ausgestalteten, stationären Riemenenden (19) des Trageriemensystems (17) zusammenwirken, wodurch das Trageriemensystems (17) an dem Rückenteil (17) reversibel gehalten ist.

8. Baukastensystem nach einem der vorhergehenden Ansprüche,
**dadurch gekennzeichnet,**
**dass** das Stützelement (23,160) wenigstens teilweise in einer Tasche (21,201) am Rückenteil (11) anordbar ist.

9. Baukastensystem nach einem der vorhergehenden Ansprüche,
**dadurch gekennzeichnet,**
**dass** durch das Kupplungsglied (28) und das Gegenkupplungsglied (48) das Brustwirbelsäulenmieder (10,200) mit dem unteren (140,120) und/oder oberen Bauchmieder (17,130) verbindbar ist.

10. Baukastensystem nach Anspruch 1, welches folgende teilweise zusätzlichen Elemente enthält:
c2) eine dorsal in dem unteren Bauchmieder (40,120) festsetzbare Basispelotte als Miederstützelement, welche entlang der Lendenwirbelsäule anordbar ist und den dritten bis zum fünften Lendenwirbel, sowie den ersten Kreuzwirbel unter Einschränkung der sagittalen Beweglichkeit stützt, wobei es sich bei der Basispelotte um eine Gliederpelotte (110) mit vier gelenkig verbundenen Gliedern (111) handelt,
d2) eine mit der Basispelotte verbindbare Lumbalerweiterung (140), welche die Basispelotte dergestalt erweitert, dass der Verband aus Basispelotte und Lumbalerweiterung (140) den ersten bis zum fünften Lendenwirbel, sowie den ersten Kreuzwirbel unter Einschränkung der sagittalen Beweglichkeit stützt, wobei die Lumbalerweiterung zwei zusätzliche Glieder, die in die bestehende Gliederpelotte eingegliedert werden, umfasst,
f2) ein an der Basispelotte festsetzbares Verbindungselement (150) zur Einschränkung der Beweglichkeit und zur Delordisierung,
g2) ein posterior wahlweise in einen Verband aus unterem Bauchmieder (40,120) und oberem Bauchmieder (17,130) oder in einen Verband aus unterem Bauchmieder (40,120) und Brustwirbelsäulenmieder (10,200) einsetzbaren Überbrückungsrahmen (170) zur Entlordosierung und Einschränkung sagittalen und frontalen Beweglichkeit im Lendenwirbelsäulenbereich, wobei der Überbrückungsrahmen (170) insbesondere am kranialen und kaudaten Ende bogenförmige Stützspangen aufweist,
h2) wobei das Stützelement (23, 160) im Wesentlichen U-förmig ist, kranial im Brustwirbelsäulenmieder (10,200) und kaudal wahlweise am Überbrückungsrahmen (170) oder am Verbindungselement (150) festsetzbar ist, und wobei die Schenkel des Stützelementes (23, 160) sich beiderseits entlang der Wirbelsäule erstrecken, um deren Aufrichtung und Entlastung in der Sagittalebene zu erzielen.

11. Baukastensystem nach Anspruch 10,
**dadurch gekennzeichnet,**
**dass** es sich bei der Basispelotte um eine Gliederpelotte (110) handelt, enthaltend vier gelenkig miteinander verbindbare Glieder (111), welche entlang der Lendenwirbelsäule angeordnet sind und den dritten bis zum fünften Lendenwirbel, sowie den ersten Kreuzwirbel transversal überbrücken.

12. Baukastensystem nach Anspruch 11,
**dadurch gekennzeichnet,**
**dass** das Baukastensystem ein Fixierelement (115) enthält, das posterior auf die Gliederpelotte (110) dergestalt aufsetzbar ist, dass Glieder (111) der Gliederpelotte (110) in Sagittalebene unbeweglich untereinander fixiert sind.

13. Baukastensystem nach Anspruch 11 oder 12,
**dadurch gekennzeichnet,**
**dass** die Lumbalerweiterung (140) für die Gliederpelotte (110) zwei gelenkig miteinander verbindbare Glieder (141) sowie zwei Stützschienen (142) enthält, wobei die Glieder (141) der Lumbalerweiterung (140) in die Gliederpelotte (110) dergestalt einsetzbar sind, dass die Glieder (111, 141) der um die Lumbalerweiterung (140) erweiterte Gliederpelotte (110) den ersten bis zum fünften Lendenwirbel, sowie den ersten Kreuzwirbel transversal überbrücken, und wobei die Stützschienen (142) lateral auf die Gliederpelotte (10) dergestalt aufsetzbar sind, dass zumindest die Glieder (111, 141), welche die ersten vier Lendenwirbel überbrücken, unbeweglich untereinander fixiert sind.

14. Baukastensystem nach einem der Ansprüche 10 bis 13,
**dadurch gekennzeichnet,**
**dass** das Baukastensystem am Überbrückungsrahmen (170) festlegbare Versteifungsstäbe (180) zur Einstellung der Steifigkeit des Überbrückungsrahmens (170) enthält.

## Claims

1. Building set for configuring thoracic spine orthoses from one or multiple elements of the building set, which comprises the following elements:
a1) lower abdomen corsage (40, 120),
b1) an upper abdomen corsage (17, 130) determinable at the cranial side of the lower abdomen corsage (40, 120),
c1) a frame-like corsage supporting element (41) determinable at the dorsal side of the lower abdomen corsage (40, 120), which is arrangeable alongside the lumbar spine and which supports the spinal column with limitation of the sagittal movement,
e1) thoracic spine corsage (10, 200) determinable at the cranial side of the lower abdomen corsage (40, 120),
g1) at least one posterior arch-like supporting clip (47), applicable alternatively in an assembly of the lower abdomen corsage (40, 120) and the upper abdomen corsage (17, 130) or in an assembly of the lower abdomen corsage (40, 120) and thoracic spine corsage (10, 200), which can be arranged at the frame-like corsage supporting element (41) and serves for delordosing and limitation in the sagittal and frontal movement,
h1) at least one supporting element (23, 160) arrangeable at the cranial side of the thoracic spine corsage (10, 200) and on the caudal side alternatively at the frame-like corsage supporting element (41, 150), which extends laterally alongside the spine column in order to achieve an erection and relieve of the sagital layer,
i1) and a abdomen pelotte (190) determinable at the ventral side of the lower abdomen corsage (40, 120) for delordosing of the lumbar vertebral column and for increasing the intra abdominal pressure.

2. Building set according to claim 1,
**characterized in that**
the supporting element (23) comprises a coupling element (28) and the corsage supporting element (41) an anti-coupling element (48), which in particular can be assembled via a form closure.

3. Building set according to claim 1 or 2,
**characterized in that**
the thoracic spine corsage (10, 200) comprises a shoulder belt system (13), with which an particularly extensive back part (11) can be hold in a backpack-like manner, and a ventral carrying strap system (17), which is also connected to the back part (11).

4. Building set according to claim 3,
**characterized in that**
the shoulder belt system (13) comprises inelastic belts (14), which particularly comprise plastic textile.

5. Building set according to claim 3 or 4,
**characterized in that**
the shoulder belt system (13) is assembled at the back part (11) using a belt path (15), wherein the belt path (15) is permanently assembled at the back part (11), particularly using elastic paths (16).

6. Building set according to one of the claims 1 to 5,
**characterized in that**
the carrying strap system (17) and/or the upper abdomen corsage (130) are reversibly attachable via mounting lobes (29) in respect to the back part (11).

7. Building set according to claim 6,
**characterized in that**
the mounting lobes (29) are in a double walled form, wherein the inner surfaces comprise hook-and-loop-fasteners, which interact with correspondingly shaped stationary edges of the strap (19) of the carrying strap system (17), whereby the carrying strap system (17) is reversibly held at the back part (11).

8. Building set according to one of the preceding claims,
**characterized in that**
the supporting element (23, 160) is at least partly arrangeable in a bag (21, 201) at the back part (11).

9. Building set according to one of the preceding claims,
**characterized in that**
the coupling element (28) and the anti-coupling element (48) of the thoracic spine corsage (10, 200) are connectable to the lower (40, 120) and/or upper abdomen corsage (17, 130).

10. Building set according to claim 1, which comprises the following, partly additional, elements:
c2) a basis pelotte determinable at the dorsal side of the lower abdomen corsage (40, 120) as a corsage supporting element, which can be assembled alongside the lumbar vertebral column and supports the third to the fifth lumbar vertebra as well as the first sacrum by constriction of the sagittal movement, wherein a basis pelotte is a joint pelotte (110) comprising four jointly connected joints (111),
d2) a basis pelotte connectable to a lumbar extension (140), which extends the basis pelotte in a way that the connection of the basis pelotte and the lumbar extension (140) supports the first to the fifth lumbar vertebra as well as the first sacrum by constriction of the sagittal movement, wherein the lumbar extension (140) comprises two additional joints, which are incorporated in the existing joint pelotte,
f2) a connecting element (150) determinable at the basis pelotte for the constriction of movement and for delordosing,
g2) a posterior bypass frame (170) alternatively applicable in a connection of lower abdomen corsage (40, 120) and upper abdomen corsage (17,130) or in a connection of lower abdomen corsage (40, 120) and thoracic spine corsage (10, 200) for delordosing and constriction of the sagittal and frontal movement in the area of the lumbar vertebra column, wherein the bypass frame (170) comprises arch-like supporting clips particularly at the cranial or caudal edge,
h2) wherein the supporting element (23, 160) is essentialy U-shaped, can be arranged cranial at the thoracic spine corsage (10, 200) and caudal alternatively at the bypass frame (170) or at the connecting element (150) and wherein the legs of the supporting element (23, 160) are reciprocally extending alongside the spinal column in order to achieve their erection and relieve in the sagital layer.

11. Building set according to claim 10,
**characterized in that**
the basis pelotte is a joint pelotte (110) comprising four jointly connectable joints (111), which are assembled alongside the lumbar vertebra column and transversally bypass the third to the fifth lumbar vertebra as well as the first sacrum.

12. Building set according to claim 11,
**characterized in that**
the building set comprises a fixation element (115), which is at the posterior side attachable to the joint pelotte (110) in a way that the joints (111) of the joint pelotte (110) are mutually, immovably fixed in the sagittal layer.

13. Building set according to claim 11 or 12,
**characterized in that**
the lumbar extension (140) comprises two jointly connectable joints (141) as well as two supporting rails (142) for the joint pelotte (110), wherein the joints (141) of the lumbar extension (140) are attachable to the joint pelotte (110) in a way that the joints (111, 141) of the joint pelotte (110) extended for the lumbar extension (140) transversely bypass the first to the fifth lumbar vertebral as well as the first sacrum and wherein the supporting rail (142) is laterally attachable to the joint pelotte (110) in a way that at least the joints (111,141) are mutually, immovably fixed, which bypass the first four lumbar vertebra.

14. Building set according to one of the claims 10 to 13,
**characterized in that**
the building set comprises fixable stiffening rods (180) at the bypass frame (170) for the adjustment of the stiffness of the bypass frame (170).

## Revendications

1. Système de construction par unités permettant de configurer une orthèse de la colonne vertébrale, composé d'un ou de plusieurs éléments du système de construction par unités qui contient les éléments suivants :
a1) une gaine abdominale inférieure (40 ; 120)
b1) une gaine abdominale supérieure (17 ; 130) pouvant être fixée en crânial au niveau de la gaine abdominale inférieure (40, 120),
c1) un élément de soutien de gaine (41) en forme de cadre pouvant être fixé en dorsal dans la gaine abdominale inférieure (40, 120) et qui peut être disposé le long de la colonne vertébrale lombaire et soutient la colonne vertébrale en limitant la mobilité sagittale,
e1) une gaine de colonne vertébrale thoracique (10, 200) pouvant être fixée en crânial au niveau de la gaine abdominale inférieure (40, 120),
g1) au moins une barrette de soutien (47) en forme d'arc, pouvant être utilisée en postérieur au choix dans une association de la gaine abdominale inférieure (40, 120) et de la gaine abdominale supérieure (17, 130) ou dans une association de la gaine abdominale inférieure (40, 120) et de la gaine de colonne vertébrale thoracique (10, 200), qui peut être disposée au niveau de l'élément de soutien de gaine (41) en forme de cadre et qui sert à soulager la lordose et à limiter la mobilité sagittale et frontale dans la zone de la colonne vertébrale lombaire,
h1) au moins un élément de soutien (23, 160) pouvant être fixé en crânial dans la gaine de colonne vertébrale thoracique (10, 200) et en caudal au choix au niveau de l'élément de soutien de gaine (41) en forme de cadre, lequel peut s'étendre latéralement le long de la colonne vertébrale pour obtenir son redressement et son soulagement dans le plan sagittal,
i1) et une pelote abdominale (190) pouvant être fixée en ventral dans la gaine abdominale inférieure (40, 120) pour soulager la lordose de la colonne vertébrale lombaire et augmenter la pression intra-abdominale.

2. Système de construction par unités selon la revendication 1, **caractérisé en ce que** l'élément de soutien (23) présente un élément d'accouplement (28) et l'élément de soutien de gaine (41) un contre-élément d'accouplement (48), lesquels peuvent être assemblés en particulier par un accouplement de forme.

3. Système de construction par unités selon la revendication 1 ou 2, **caractérisé en ce que** la gaine de colonne vertébrale thoracique (10, 200) présente un système de ceinture d'épaule (13) par lequel élément de dos (11), en partie de grande surface, est maintenu à la façon d'un sac à dos, ainsi qu'un système de courroie de soutien ventral (17) qui est également relié à l'élément de dos (11).

4. Système de construction par unités selon la revendication 3, **caractérisé en ce que** le système de ceinture d'épaule (13) contient des ceintures non élastiques (14) qui présentent en particulier un textile synthétique.

5. Système de construction par unités selon la revendication 3 ou 4, **caractérisé en ce que** le système de ceinture d'épaule (13) est disposé au niveau de l'élément de dos (11) par l'intermédiaire d'éléments de fixation de ceinture (15), les éléments de fixation de ceinture (15) étant fixés de façon non amovible à l'élément de dos (11), en particulier par des bandes élastiques (16).

6. Système de construction par unités selon l'une des revendications 1 à 5, **caractérisé en ce que** le système de courroie de soutien (17) ou la gaine abdominale supérieure (130) peut être fixé(e) de façon réversible à l'élément de dos (11) par des pattes de fixation (29).

7. Système de construction par unités selon la revendication 6, **caractérisé en ce que** les pattes de fixation (29) sont exécutées à double paroi, les faces internes étant munies de fermetures velcro qui collaborent avec des extrémités de courroie (19), fixes et formées en conséquence, du système de courroie de soutien (17), le système de courroie de soutien (17) étant maintenu de façon réversible au niveau de l'élément de dos (17).

8. Système de construction par unités selon l'une des revendications précédentes, **caractérisé en ce que** l'élément de soutien (23, 160) peut être disposé au moins partiellement dans une poche (21, 201) au niveau de l'élément de dos (11).

9. Système de construction par unités selon l'une des revendications précédentes, **caractérisé en ce que** la gaine de colonne vertébrale thoracique (10, 200) peut être reliée à la gaine abdominale inférieure (140, 120) et/ou supérieure (17, 130) par l'élément d'accouplement (28) et le contre-élément d'accouplement (48).

10. Système de construction par unités selon la revendication 1, qui contient les éléments partiellement supplémentaires suivants :
c2) une pelote de base pouvant être fixée en dorsal dans la gaine abdominale inférieure (40, 120) en tant qu'élément de soutien de gaine, qui peut être disposée le long de la colonne vertébrale lombaire et soutient les troisième à cinquième vertèbres lombaires, ainsi que la première vertèbre sacrée, en limitant la mobilité sagittale, où il s'agit concernant la pelote de base d'une pelote à éléments (110) comportant quatre éléments (111) reliés par articulation,
d2) une extension lombaire (140) pouvant être reliée à la pelote de base, qui élargit la pelote de base de façon telle que l'association de la pelote de base et de l'extension lombaire (140) soutient les première à cinquième vertèbres lombaires ainsi que la première vertèbre sacrée en limitant la mobilité sagittale, l'extension lombaire comportant deux éléments supplémentaires qui sont intégrés dans la pelote à éléments existante,
f2) un élément de liaison (150) pouvant être fixé à la pelote de base afin de limiter la mobilité et de soulager la lordose,
g2) un cadre de pontage (170) pouvant être utilisé en postérieur au choix dans une association de la gaine abdominale inférieure (40, 120) et de la gaine abdominale supérieure (17, 130) ou dans une association de la gaine abdominale inférieure (40, 120) et de la gaine de colonne vertébrale thoracique (10, 200) afin de soulager la lordose et de limiter la mobilité sagittale et frontale dans la zone de la colonne vertébrale lombaire, le cadre de pontage (170) présentant en particulier en extrémité crâniale et caudale des barrettes de soutien en forme d'arc,
h2) l'élément de soutien (23, 160) étant essentiellement en forme de U, pouvant être fixé en crânial dans la gaine de colonne vertébrale thoracique (10, 200) et en caudal au choix au niveau du cadre de pontage (170) ou au niveau de l'élément de liaison (150), et les branches de l'élément de soutien (23, 160) s'étendant des deux côtés le long de la colonne vertébrale afin d'atteindre son redressement et un soulagement dans le plan sagittal.

11. Système de construction par unités selon la revendication 10, **caractérisé en ce qu'**il s'agit concernant la pelote de base d'une pelote à éléments (110) contenant quatre éléments (111) pouvant être reliés entre eux par articulation, et qui sont disposés le long de la colonne vertébrale lombaire et pontent de façon transversale les troisième à cinquième vertèbres lombaires ainsi que la première vertèbre sacrée.

12. Système de construction par unités selon la revendication 11, **caractérisé en ce que** le système de construction par unités contient un élément de fixation (115) qui peut être appliqué en postérieur sur la pelote à éléments (110) de façon telle que les éléments (111) de la pelote à éléments (110) sont fixés entre eux de façon immobile dans le plan sagittal.

13. Système de construction par unités selon la revendication 11 ou 12, **caractérisé en ce que** l'extension lombaire (140) de la pelote à éléments (110) contient deux éléments (141) pouvant être reliés entre eux par articulation ainsi que deux rails de soutien (142), les éléments (141) de l'extension lombaire (140) pouvant être introduits dans la pelote à éléments (110) de façon à ce que les éléments (111, 141) de la pelote à éléments (110) agrandie de l'extension lombaire (140) pontent transversalement les première à cinquième vertèbres lombaires ainsi que la première vertèbre sacrée, et où les rails de soutien (142) peuvent être appliqués latéralement sur la pelote à éléments (110) de façon telle qu'au moins les éléments (111, 141) qui pontent les quatre premières vertèbres lombaires soient fixés entre eux de façon immobile.

14. Système de construction par unités selon l'une des revendications 10 à 13, **caractérisé en ce que** le système de construction par unités contient au niveau du cadre de pontage (170) des tiges de renforcement (180) pouvant être bloquées et permettant de régler la rigidité du cadre de pontage (170).
